# FASCICULE DE BREVET EUROPEEN

(11) **EP 3 439 726 B1**
(45) Date de publication et mention de la délivrance du brevet: **09.06.2021**
(21) Numéro de dépôt: 17720858.4
(22) Date de dépôt: 07.04.2017
(51) Int. Cl.: A61M 25/00, A61M 25/10

(54) **DISPOSITIF HÉMOSTATIQUE POUR LE TRAITEMENT DU SAIGNEMENT POSTOPÉRATOIRE DE LA CAVITÉ PROSTATIQUE APRÈS CHIRURGIE DE L'HYPERTROPHIE BÉNIGNE DE LA PROSTATE**
HÄMOSTATISCHE VORRICHTUNG ZUR BEHANDLUNG VON POSTOPERATIVER BLUTUNG DER PROSTATAHÖHLE NACH DER OPERATIVEN BEHANDLUNG VON BENIGNER PROSTATAHYPERTROPHIE
HAEMOSTATIC DEVICE FOR TREATING POSTOPERATIVE BLEEDING OF THE PROSTATIC CAVITY AFTER BENIGN PROSTATIC HYPERTROPHY SURGERY

(30) Priorité: 08.04.2016 FR 1653112
(43) Date de publication de la demande: 13.02.2019
(73) Titulaire: Devonec, Marian, 01700 Miribel (FR)
(72) Inventeur: Devonec, Marian, 01700 Miribel (FR)
(74) Mandataire: Chevalier, Renaud Philippe
(86) Numéro de dépôt international: PCT/FR2017/050841
(87) Numéro de publication internationale: WO 2017/174950

(56) Documents cités:
- WO-A1-99/07420
- WO-A1-2010/079480
- DE-A1-102011 110 778
- FR-A- 1 190 606
- FR-A1- 2 754 701
- US-A- 4 660 560
- US-A- 4 705 502
- US-A1- 2002 165 521
- US-A1- 2004 059 290
- US-A1- 2012 035 595

## Description

La présente invention concerne un dispositif hémostatique après chirurgie de l'hypertrophie bénigne de la prostate. A côté de cette application, d'autres d'applications à d'autres maladies de la prostate ou d'autres organes du corps humain sont possibles.

L'hypertrophie bénigne ou adénome de la prostate correspond à la croissance des glandes péri-urétrales de la zone de transition située de chaque côté du segment prostatique du canal de l'urètre masculin. Le grossissement ou hypertrophie de la zone de transition comprime le segment prostatique de l'urètre. Cette compression du canal gène l'évacuation de l'urine de la vessie. Après épuisement des effets du traitement médical, la chirurgie est proposée. La chirurgie consiste à enlever l'urètre prostatique et le tissu hypertrophié qui entoure l'urètre et le comprime. La chirurgie supprime le tissu hypertrophié, elle fait disparaître la compression du canal et élargit de façon significative le passage à travers la prostate. La cavité creusée à l'intérieur de la prostate remplace l'urètre prostatique comprimé. L'évacuation de l'urine de la vessie se fait à travers la cavité et devient facile. La chirurgie est pratiquée le plus souvent par les voies naturelles (chirurgie endoscopique ou résection endo-urétrale), plus rarement par une incision cutanée au-dessus du pubis (chirurgie ouverte ou adénomectomie).

La chirurgie endoscopique de l'hypertrophie bénigne de la prostate est l'intervention la plus couramment pratiquée en chirurgie urologique. La variation et l'imprédictibilité de la durée de l'hospitalisation est la conséquence directe de la variation et imprédictibilité du saignement après chirurgie. La condition sine qua non du contrôle de la durée de séjour et de son raccourcissement est le contrôle du saignement après chirurgie.

Toute technique chirurgicale est responsable d'un saignement de la cavité créée par l'acte chirurgical au centre de la prostate. L'importance du saignement et sa durée sont variables et imprévisibles.

La glande prostatique a une alimentation vasculaire importante. Le saignement existe après toute chirurgie prostatique mais varie selon la technique chirurgicale utilisée (la chirurgie ouverte par voie sus-pubienne trans-vésicale (opération de Freyer) et trans-capsulaire (opération de Millin), la chirurgie endoscopique, c'est-à-dire, la résection par courant électrique de copeaux de prostate, la vaporisation par laser KTP (potassium-titanyl-phosphate), l'énucléation par laser Holmium ou Thulium.

Le saignement vient de l'effet de coupe de l'instrument au niveau de
- la muqueuse urétrale des deux orifices opposés de la cavité prostatique : au niveau de l'orifice de la vessie, au niveau de la section de l'urètre, à l'apex de la prostate situé juste au-dessus du sphincter strié urinaire,
- le saignement provient également de la surface de la cavité prostatique, de la blessure de vaisseaux artériels ou veineux de taille intermédiaire, et du saignement en nappe venant des capillaires et vaisseaux de petite taille coupés à la surface de la cavité.

Après l'opération la cavité prostatique est largement ouverte du côté de la vessie. De ce fait le saignement s'écoule vers la vessie à travers la découpe large de l'orifice de la vessie réalisée pendant l'opération. Cette large découpe de l'orifice de la vessie est nécessaire pour permettre la migration du liquide d'irrigation et chasser les copeaux de tissu prostatique réséqués vers l'intérieur de la vessie. L'irrigation est nécessaire en permanence du début à la fin du geste de résection. L'irrigation permet de diluer le sang et d'éclaircir le champ de vision de l'opérateur.

Dès la fin de l'opération, une sonde de Foley est mise en place dans l'urètre et son ballonnet est gonflé dans la vessie. La sonde permet le drainage de l'urine, et du liquide d'irrigation. L'irrigation de la vessie par du sérum physiologique est systématiquement débutée immédiatement après mise en place de la sonde. L'irrigation est nécessaire parce qu'un saignement persiste après la fin de la chirurgie et surtout peut se réactiver dans les heures ou les jours suivants. Le liquide d'irrigation dilue le sang et prévient la formation de caillots à l'intérieur de la vessie. Malgré l'utilisation de ce mode de prévention, des caillots peuvent se former à l'intérieur de la vessie et venir obstruer les orifices de drainage situés à l'extrémité distale de la sonde.

Chez le patient opéré, le saignement et l'obstruction de sa sonde à demeure par les caillots l'exposent à un triple risque : une rétention aiguë d'urine très douloureuse par distension excessive de sa vessie ; un risque de contamination bactérienne et un risque de transfusion sanguine en cas de saignement important.

Pour l'infirmière le saignement impose une surveillance permanente de la couleur du liquide drainé par la sonde à demeure. Cette surveillance et les manipulations relatives (réglage du débit de l'irrigation, décaillotage vésical) sont également consommatrices de temps de travail.

Le contrôle du saignement peut être obtenu de deux façons au cours de la chirurgie et après chirurgie de l'hypertrophie bénigne de la prostate. L'hémostase peut être obtenue par une action focale et une action globale sur la vascularisation de la prostate. L'action focale consiste à coaguler pendant l'opération avec un instrument chaque vaisseau qui saigne. L'action globale permet après l'opération d'agir sur l'ensemble des vaisseaux de la paroi de la cavité prostatique.

Après l'opération, deux moyens d'action globale sont utilisés en routine, l'irrigation vésicale et la traction sur la sonde de Foley. L'irrigation de liquide par la sonde de Foley dilue le sang et prévient la formation de caillots à l'intérieur de la vessie, mais contribue seulement indirectement à l'hémostase. A l'opposé deux moyens favorisent directement l'hémostase, la traction sur la sonde de Foley et l'utilisation d'un agent thérapeutique. La traction sur la sonde de Foley est une technique utilisée mais non standardisée.

L'inconvénient majeur de cette technique est son absence de reproductibilité : non seulement la force de traction exercée sur la sonde de Foley n'est pas standardisée, mais la cible de la force n'est pas standardisée non plus. La cible dépend de la position du ballonnet de la sonde par rapport à la vessie ou la prostate. Les caractéristiques du ballonnet ne sont pas standardisées. Le mode de traction et de fixation de la sonde ne sont pas standardisés: le corps de la sonde est souvent collé sur la cuisse ou sur le pubis du patient avec un morceau de sparadrap placé en travers du corps de la sonde. La traction peut être exercée sur la sonde par une cordelette attachée d'un côté à la sonde et du côté opposé à un poids suspendu au pied du lit équipé d'une potence et d'une poulie supportant la corde.

L'élasticité du corps de la sonde longue de 40 cm et la pression appliquée par le ballonnet à l'extrémité distale de la sonde sont inconnues et varient avec la sonde utilisée. De plus la tension exercée au niveau de l'extrémité proximale de la sonde à l'extérieur du patient n'est transmise que partiellement au ballonnet à l'extrémité opposée de la sonde, du fait d'une perte d'énergie liée au coude à angle droit de l'urètre entre sa portion bulbaire et sa portion prostatique. L'élasticité et la forme du ballonnet ne sont pas standardisées. Les dimensions du ballonnet ne sont pas standardisées. Le volume de remplissage du ballonnet n'est pas standardisé et le diamètre du corps du cathéter varie de 18 à 24 CH (charrière). Ce choix du diamètre du cathéter, du volume de remplissage du ballonnet est opérateur dépendant. Le choix du cathéter et la façon dont l'opérateur l'utilise ne repose sur aucun critère standardisé mais sur l'expérience personnelle et empirique de chaque opérateur.

La cible de la force exercée par le cathéter n'est pas standardisée. L'opinion des Urologues varie sur la position idéale du ballonnet; pour certains le ballonnet doit être dans la cavité prostatique et de ce fait en contact avec le versant interne de la cavité qui saigne et en contact avec l'orifice inférieur de la cavité, c'est à dire avec le sphincter strié de l'urètre ; pour d'autres il doit être dans la vessie et en contact avec l'orifice de la vessie, c'est-à-dire avec l'orifice supérieur de la cavité prostatique.

L'usage d'un agent thérapeutique a été essayé pour améliorer l'hémostase à l'aide de l'injection d'un agent hémostatique voire même d'un agent fibrinolytique à l'intérieur de la cavité prostatique ou de la vessie. Parmi les agents hémostatiques, la thrombine, la fibrine, la gélatine ont été essayées ; parmi les agents fibrinolytiques, l'acide amino-caproïque a été essayé.

Ces agents hémostatiques appartiennent à l'une des différentes classes suivantes : hémostatiques topiques, dérivés de la fibrine, hémostatiques matriciels, agents fibrinolytiques. Aucun d'eux n'est utilisé régulièrement pour le contrôle de l'hémostase après chirurgie de l'hypertrophie bénigne de la prostate.

Parmi les agents de coagulation topiques : la cellulose, la gélatine, la thrombine, le collagène, la fibrine, le fibrinogène ont été proposés. Parmi les agents anti-fibrinolytiques : l'acide amino-caproïque, l'acide tranexamique, l'aprotinine ont été proposés. Plus récemment, les agents de comblement à base de fibrine ont été proposés. Un des inconvénients d'un grand nombre de ces agents est leur origine humaine ou animale avec un risque intrinsèque de contamination par des virus ou des particules de protéines chez le receveur. Ces agents interfèrent avec la coagulation sanguine naturelle et peuvent provoquer une coagulation excessive localement ou à distance. Par ailleurs, le caillot évolue naturellement vers sa lyse. La fibrinolyse naturelle du caillot libère des facteurs fibrinolytiques qui peuvent réactiver et entretenir le saignement. Plus le caillot est petit, plus ce risque lié à la fibrinolyse diminue et meilleure sera l'hémostase.

A ce jour, le saignement postopératoire est imprévisible et traité par irrigation vésicale, selon une attitude passive et empirique, consistant en la dilution du sang provenant de la blessure des vaisseaux de la cavité prostatique dont l'hémostase n'est pas contrôlée. L'irrigation vésicale est contreproductive : elle dilue et élimine les facteurs de coagulation; elle s'oppose à la formation du caillot et au processus d'hémostase naturelle.

L'usage de cathéter pour contrôler le saignement a été proposé dans plusieurs domaines.

En Urologie, le cathéter ou sonde de Foley à trois voies est systématiquement utilisé pour le contrôle de l'hémostase après chirurgie de l'hypertrophie bénigne de la prostate. Les trois voies ou autrement dit les trois canaux se répartissent en un premier canal pour gonfler le ballonnet situé à l'extrémité distale de la sonde et destiné à son maintien en place à l'intérieur de la vessie ; un deuxième canal central pour le drainage de l'urine, et un troisième canal pour l'irrigation de la vessie avec un liquide d'irrigation.

En gynécologie, un cathéter hémostatique a été proposé pour le contrôle du saignement de l'utérus après accouchement et expulsion du placenta par Claren et Ulmsten (PCT application : WO 97/27810). Le cathéter est inséré à l'intérieur de la cavité utérine et le ballonnet est gonflé à l'intérieur de la cavité. Une traction sur le cathéter permet de fermer de l'intérieur l'orifice de la cavité utérine. Le ballonnet stoppe l'écoulement du sang en dehors de la cavité, mais n'empêche pas le remplissage de la cavité par le sang qui provient de la zone où était inséré le placenta.

Dans le domaine cardio-vasculaire, le cathéter de Gillot est un cathéter borgne à son extrémité distale avec deux ballonnets en position fixe sur le cathéter, et distants l'un de l'autre de 10 cm environ. Le positionnement de ce cathéter dans un gros vaisseau comme l'aorte, suivi du gonflage des deux ballonnets permet l'occlusion de la lumière du vaisseau, arrête la circulation du sang et isole le segment de vaisseau situé entre les deux ballonnets. Un orifice situé dans la paroi du corps du cathéter entre les deux ballonnets permet la perfusion d'une solution de protection à basse température à l'intérieur des vaisseaux naissant du segment isolé de l'aorte par exemple ainsi que de l'organe ou des organes alimentés par ces vaisseaux.

Le dispositif décrit par Palasis (US2004/0059290 A1) consiste en un cathéter avec deux ballonnets en position fixe permettant d'isoler un segment de vaisseau et d'injecter un agent thérapeutique dans ce segment de vaisseau. La particularité du dispositif repose sur la présence d'un revêtement sur la paroi des ballonnets dans le but d'éviter l'abrasion de l'endothélium au niveau de la zone de contact et de forte pression entre le ballonnet et la paroi du vaisseau.

Le dispositif décrit par Ronsse (FR2 803 532) consiste en un cathéter avec deux ballonnets en position fixe permettant d'isoler un segment de vaisseau tout en préservant un léger flux sanguin grâce un orifice situé sur le cathéter en amont du ballonnet proximal ; l'écoulement sanguin à travers le cathéter sort par l'extrémité distale du cathéter.

Un autre type de cathéter est décrit dans la demande de brevet Dietrich EP2110151A1 avec deux ballonnets non plus en position fixes comme décrit plus haut, mais incorporés à des corps coaxiaux mobiles permet une adaptation précise de la longueur du segment occlus et isolé, à la longueur du segment de vaisseau à traiter. Des repères radio-opaques incorporés aux ballonnets permettent le positionnement relatif des ballonnets sous fluoroscopie.

En Gastro-entérologie, un cathéter avec deux ballonnets dont la position est fixe a été conçu par Zhang (CN201500154) pour isoler et traiter un segment du tube digestif. Après positionnement des deux ballonnets de part et d'autre de la zone où le saignement est observé, les ballonnets sont gonflés dans la lumière du tube digestif par exemple à la hauteur de l'œsophage et de l'estomac. L'augmentation de la pression à l'intérieur du segment isolé diminue le saignement par tamponnade du segment isolé. Le document US2002/0165521 décrit un dispositif hémostatique selon le préambule de la revendication 1.

L'ensemble de ces dispositifs permet l'occlusion d'un segment de la lumière d'un vaisseau u d'un tube naturel comme le tube digestif. L'occlusion est obtenue par le gonflage des ballonnets de part et d'autre de la zone à traiter. L'occlusion de la lumière provient de façon directe des ballonnets qui exercent des forces dirigées de façon centrifuge radiaire et perpendiculaire à l'axe de la lumière du vaisseau ou du tube naturel. Les forces sont générées de l'intérieur de la lumière et sont appliquées de façon perpendiculaire contre la paroi du vaisseau ou du tube naturel. Elles augmentent le diamètre de la lumière du vaisseau ou du tube naturel traité. L'ensemble de ces dispositifs ne permettent pas le contrôle du saignement après chirurgie de l'hypertrophie bénigne de la prostate. Ces dispositifs ne permettent pas l'occlusion d'une cavité creusée dans un segment de tube naturel (tel que l'urètre prostatique) à sa jonction avec une cavité naturelle (telle que la vessie). Après chirurgie de la prostate, le simple gonflage d'un ou de deux ballonnets exerçant de façon directe une force contre la paroi de la cavité prostatique ne permet pas d'obtenir l'occlusion de la cavité. En effet, les forces centrifuges radiaires perpendiculaires à l'axe longitudinal de la cavité prostatique, générées de l'intérieur de la lumière et appliquées de façon perpendiculaire à la paroi de la cavité sont inefficaces et contreproductives ; ces forces ne permettent pas l'occlusion de la cavité prostatique et ne font qu'augmenter le saignement en élargissant la taille des brèches vasculaires au niveau de la paroi de la cavité.

L'invention a pour but de remédier à tout ou partie des inconvénients précités, afin d'obtenir une hémostase immédiate et définitive, dès la fin de la chirurgie, au bloc opératoire après toute technique chirurgicale de traitement de l'hypertrophie bénigne de la prostate.

L'invention est décrite dans le set de revendications attaché à la description. L'invention a pour objet un dispositif hémostatique pour le traitement du saignement postopératoire de la cavité prostatique comportant au moins un cathéter urétral comprenant :
- un corps comportant un premier canal,
- un premier ballonnet gonflable ménagé sur ledit corps, le premier ballonnet étant en connexion fluidique avec le premier canal, le premier ballonnet étant destiné à être placé dans la vessie contre le col de la vessie et étant configuré pour exercer une pression déterminée à l'état gonflé sur la cavité prostatique de manière à obturer et isoler la cavité prostatique, à réduire le volume de la cavité prostatique et à occlure les vaisseaux de la paroi de la cavité prostatique,
caractérisé en ce que le dispositif hémostatique comprend en outre au moins un dispositif de blocage de position configuré pour figer la position et la tension du cathéter dans l'urètre, le dispositif de blocage étant relié directement ou indirectement au cathéter.

Le dispositif hémostatique selon l'invention, permet d'isoler et d'obturer la cavité prostatique au moyen notamment du premier ballonnet tout en permettant l'hémostase des vaisseaux de la paroi de la cavité prostatique grâce leur compression sous l'effet du ballonnet. Cette invention permet la réduction du volume du saignement au volume de la cavité prostatique et l'absence de saignement postopératoire en dehors de ladite cavité prostatique obturée. L'invention évite la mise en place d'une sonde de Foley avec irrigation de liquide et élimine ainsi chez le patient les différents risques de contaminations et de complications liées à ladite sonde et à l'irrigation. En outre, le dispositif hémostatique selon l'invention, présente également des avantages pour le personnel hospitalier.

Le dispositif hémostatique selon l'invention, permet la réduction de la charge de travail de l'infirmière: il n'y a plus besoin de renouveler les sacs de liquide d'irrigation, pas besoin de manœuvres de décaillotage de la vessie ; il y a suppression de la surveillance de la couleur du liquide de lavage pour adapter le débit de l'irrigation à la couleur de l'effluent mélange d'urine et de liquide de lavage drainé par la sonde. Cette surveillance chronophage est supprimée.

En outre, le dispositif hémostatique permet la réduction des coûts liés à la consommation des sacs de liquide d'irrigation, de petits matériels jetables, du coût lié au temps infirmier pour la surveillance de l'irrigation et du coût lié au traitement des liquides biologiques contaminés par du sang. Une procédure ambulatoire de la chirurgie de l'hypertrophie bénigne de la prostate réduirait la durée de l'hospitalisation de 4 à 8 fois et le coût des soins dans les mêmes proportions. Une réduction des coûts liés à l'utilisation d'antibiotiques est également attendue, puisqu'une infection urinaire iatrogène est systématiquement observée après 3-4 jours de cathétérisme par sonde de Foley. La réduction du sondage de 4-8 jours à une seule journée diminue le risque d'infection et l'usage d'antibiotiques de façon très significative. Le risque de sélection de bactéries résistantes aux antibiotiques est également diminué.

Selon une caractéristique de l'invention, la standardisation du dispositif hémostatique est obtenue grâce à un organe de blocage standardisé configuré de façon à fixer la position et la tension du cathéter dans l'urètre et à exercer contre la cavité prostatique une pression déterminée et répétable par l'intermédiaire du corps du cathéter soumis à une force de traction déterminée et répétable de l'ordre de 40 Newtons.

Selon une caractéristique de l'invention, le dispositif de blocage est relié directement au cathéter ou indirectement par un moyen de raccordement.

Selon une caractéristique de l'invention, la pression déterminée est une pression reproductible.

Selon une caractéristique de l'invention, le corps du cathéter présente un diamètre externe de l'ordre de 22CH.

Selon une caractéristique de l'invention, la longueur du cathéter est de l'ordre de 40 cm.

Préférentiellement, le cathéter est inextensible de façon à transmettre au ballonnet une force de traction déterminée et répétable. Le matériau du corps du cathéter est préférentiellement le nylon.

Avantageusement, le corps présente une flexibilité, comparable à celui d'une sonde de Foley, permettant une insertion facile du cathéter dans l'urètre masculin.

Selon une caractéristique de l'invention, le premier ballonnet présente un volume à gonfler invariable, ce qui permet de s'assurer de la standardisation et de la répétabilité du gonflage. Le gonflage permet de sécuriser le premier ballonnet à l'intérieur de la vessie, et d'empêcher le glissement du premier ballonnet vers la cavité prostatique. Ainsi, en tirant sur le premier ballonnet par l'intermédiaire du corps du cathéter, la cavité prostatique est d'une part écrasée et obturée et d'autre part les vaisseaux de la paroi de la cavité prostatique sont comprimés et obturés. Cette compression des vaisseaux diminue ainsi le saignement. L'effet de compression exercé par le ballonnet est standardisé. Le gonflage à haute pression du ballonnet rigidifie sa paroi et lui confère une forme standardisée.

Selon l'invention, la position correcte du premier ballonnet du cathéter doit être à l'extérieur de la cavité prostatique, c'est-à-dire, à l'intérieur de la vessie et plus particulièrement au niveau de l'orifice de la vessie, c'est-à-dire, l'orifice supérieur de la cavité prostatique. La compression des vaisseaux de la paroi de la cavité de la prostate par le ballonnet placé dans la vessie et comprimant la cavité prostatique par le dessus est plus efficace que l'action inverse, c'est-à-dire, l'étirement des vaisseaux de la paroi de la cavité prostatique par le gonflage du ballonnet placé à l'intérieur de ladite cavité. L'étirement des vaisseaux de la paroi augmente la taille des brèches vasculaires et augmente le saignement.

Selon une caractéristique de l'invention, le premier ballonnet présente une forme globalement cylindrique, à l'état gonflé. Avantageusement, la forme du premier ballonnet est invariable même lorsqu'il est exposé à des forces extérieures telles qu'une traction opérée sur l'extrémité proximale du cathéter, et ce malgré l'opposition d'une force de résistance appliquée à la base du premier ballonnet.

Selon une caractéristique de l'invention, le premier ballonnet comprend une base destinée à être en contact avec l'orifice supérieur de la cavité prostatique, lorsque le dispositif hémostatique est mis en place.

Selon une autre caractéristique de l'invention, la base du premier ballonnet est sensiblement plane, ce qui permet une occlusion étanche de l'orifice supérieur de la cavité prostatique. Avantageusement, la forme de la base du premier ballonnet empêche l'engagement du ballonnet à l'intérieur de la cavité prostatique et garantit ainsi une compression efficace de la cavité prostatique.

Selon une caractéristique de l'invention, la paroi du premier ballonnet est conçue pour résister à une haute pression. On entendra par haute pression une pression sensiblement égale ou supérieure à 2Bar .

Selon une caractéristique de l'invention, le matériau de la paroi est préférentiellement le nylon.

Selon une caractéristique de l'invention, la paroi du premier allonnet est recouverte sur au moins la moitié inférieure de sa surface, en contact avec l'orifice supérieur de la cavité prostatique, par un revêtement hydrophile facilitant l'étanchéité de la cavité prostatique.

Selon une caractéristique de l'invention, le corps comprend au moins un deuxième canal, un deuxième ballonnet, ledit deuxième canal étant relié fluidiquement au deuxième ballonnet, le deuxième ballonnet étant conformé pour à l'état gonflé s'étendre dans la cavité prostatique.

Selon une caractéristique de l'invention, le cathéter comporte un troisième canal comprenant un orifice de sortie, et un orifice d'entrée, le troisième canal étant configuré pour acheminer depuis l'orifice d'entrée un agent thérapeutique vers l'orifice de sortie, l'orifice de sortie étant destiné à déboucher dans la cavité prostatique en état d'utilisation.

Selon une caractéristique de l'invention, l'agent thérapeutique est un ensemble de peptides de synthèse auto-agrégeant ou un agent coagulant ou un agent de comblement.

Préférentiellement, l'agent thérapeutique appartient à l'une des classes suivantes d'agents qui peuvent être utilisés seul ou en combinaison : les agents alpha-mimétiques ou les agents coagulants ou les agents embolisants ou les agents sclérosants ou les agents de remplissage ou de comblement ou un agent de la classe des peptides auto-agrégeant ou tout autre agent facilitant l'hémostase et la cicatrisation.

Selon une caractéristique de l'invention, l'agent thérapeutique se présente préférentiellement sous la forme d'un gel dont la viscosité est supérieure à celle d'une solution d'irrigation classiquement utilisée. L'agent idéal doit favoriser l'occlusion des vaisseaux macroscopiquement, microscopiquement, voire même à l'échelle nanométrique. L'agent idéal doit être de préférence indépendant de la cascade de coagulation naturelle de façon à éviter les risques liés à la lyse naturelle du caillot décrits plus haut.

Selon une caractéristique de l'invention, le dispositif hémostatique comprend au moins un quatrième canal, un troisième ballonnet, ledit quatrième canal étant relié fluidiquement au troisième ballonnet, le troisième ballonnet étant conformé pour à l'état gonflé s'étendre dans l'urètre bulbaire.

Selon une caractéristique de l'invention, le troisième ballonnet est ménagé sur le dispositif de blocage du dispositif hémostatique.

Selon une caractéristique de l'invention, la paroi du premier ballonnet et/ou du deuxième ballonnet présente un revêtement hydrophile sur sa surface extérieure.

Selon une caractéristique de l'invention, le corps comprend un canal de drainage d'urine.

Selon une caractéristique de l'invention, le corps comprend un canal d'irrigation d'un liquide d'irrigation.

Selon une caractéristique de l'invention, l'ensemble des canaux du cathéter sont agencés concentriquement dans le corps du cathéter.

Selon une caractéristique de l'invention, le dispositif de blocage est attaché à un moyen d'arrimage ou au cathéter.

L'invention sera mieux comprise, grâce à la description ci-après, qui se rapporte à des modes de réalisation selon la présente invention, donnés à titre d'exemple non limitatifs et expliqués avec référence aux dessins schématiques annexés, dans lesquels:
- la figure 1 est une vue de dessus d'un dispositif hémostatique selon un premier mode de réalisation,
- la figure 2 est une vue de dessus du dispositif hémostatique représenté en figure 1 selon une première variante du premier mode de réalisation,
- la figure 3 est une vue de dessus du dispositif hémostatique selon l'invention selon un deuxième mode de réalisation,
- la figure 4 est une vue de dessus du dispositif hémostatique selon l'invention représenté en figure 3 selon une première variante du deuxième mode de réalisation,
- la figure 5 est une vue de dessus du dispositif hémostatique représenté en figure 4 et selon une découpe segmentaire M, N, O,
- la figure 6 est une vue de dessus du dispositif de blocage du dispositif hémostatique selon un premier exemple de réalisation,
- la figure 7A est une vue de dessus du dispositif hémostatique selon une première variante du deuxième mode de réalisation représenté en figure 4 avec les ballonnets en état gonflé,
- les figures 7B à 7F sont des vues en coupe transversale dudit dispositif représenté en figure 7A selon les axes B-B, C-C, D-D, E-E, FF,
- la figure 8 est une vue en coupe longitudinale d'un premier segment M du dispositif hémostatique selon une première variante du deuxième mode de réalisation représenté en figure 5,
- la figure 9 est une vue en coupe longitudinale d'un deuxième segment N du dispositif hémostatique selon une première variante du deuxième mode de réalisation représenté en figure 5,
- la figure 10 est une vue en coupe longitudinale d'un troisième segment O du dispositif hémostatique selon une première variante du deuxième mode de réalisation représenté en figure 5,
- La figure 11 est une vue de dessus du dispositif hémostatique selon une deuxième variante du deuxième mode représenté en figure 4, la deuxième variante étant caractérisée par un dispositif de blocage selon un deuxième exemple de réalisation,
- la figure 12 est une vue de dessus du dispositif hémostatique représenté en figure 11, comprenant un dispositif de blocage selon une variante du deuxième exemple de réalisation du dispositif de blocage,
- la figure 13 est une vue de dessus du dispositif hémostatique selon un troisième mode de réalisation,
- la figure 14 est une vue de dessus du dispositif hémostatique représenté en figure 13, et selon une découpe segmentaire M, N, O,
- les figures 15 à 17 sont des vues en coupe du dispositif hémostatique selon le troisième mode de réalisation et selon les segments M, N, O représentés en figure 14,
- la figure 18 est une vue de dessus du dispositif hémostatique selon une variante du troisième mode de réalisation représenté en figure 13 comprenant un dispositif de blocage selon le deuxième exemple de réalisation,
- la figure 19 est une vue de dessus du dispositif hémostatique représenté en figure 18, comprenant un dispositif de blocage selon une variante du deuxième exemple de réalisation du dispositif de blocage,
- la figure 20A est une vue schématique des différents organes de blocage du dispositif de blocage selon le deuxième exemple de réalisation,
- la figure 20B est une vue schématique des différents organes de blocage du dispositif de blocage selon la variante du deuxième exemple de réalisation,
- la figure 21 est une vue sagittale de l'appareil uro-génital masculin avant chirurgie,
- la figure 22 est une vue sagittale de l'appareil uro-génital masculin après chirurgie,
- les figures 23 à 26 sont des vues sagittales de l'appareil uro-génital masculin, aux différentes étapes d'insertion et d'activation du dispositif hémostatique selon le premier mode de réalisation représenté en figure 1,
- la figure 27 est une vue de face de l'appareil uro-génital masculin après chirurgie avant activation du dispositif hémostatique selon la première variante du deuxième mode de réalisation représenté en figure 4 et 7A,
- la figure 28 est une vue de face de l'appareil uro-génital masculin après chirurgie après activation du dispositif hémostatique représenté en figure 27,
- la figure 29 est une vue de face de l'appareil uro-génital masculin après chirurgie après activation du dispositif hémostatique représenté en figure 27, et après instillation de l'agent thérapeutique dans la cavité prostatique,
- les figures 30 et 31 illustrent l'effet de la compression et de la plicature de la paroi prostatique sur les vaisseaux qui la traversent et en conséquence l'effet de la compression sur lesdits vaisseaux et sur le contrôle mécanique de l'hémostase,
- la figure 32 est une vue de face de l'appareil uro-génital masculin après chirurgie avant activation du dispositif hémostatique représenté figure 11, c'est-à-dire selon une deuxième variante du deuxième mode de réalisation, la deuxième variante étant caractérisée par le deuxième exemple du dispositif de blocage non représenté sur la figure 32,
- la figure 33 est une vue de face de l'appareil uro-génital masculin après chirurgie après activation du dispositif hémostatique selon la figure 32
- la figure 34 est une vue de face de l'appareil uro-génital masculin après chirurgie après activation du dispositif hémostatique selon la figure 32, et après instillation de l'agent thérapeutique dans la cavité prostatique,
- la figure 35 est une vue de face de l'appareil uro-génital masculin après chirurgie avant activation du dispositif hémostatique représenté figure 18, c'est-à-dire selon une variante du troisième mode de réalisation, ladite variante étant caractérisée par le deuxième exemple de dispositif de blocage non représenté sur la figure 35,
- la figure 36 est une vue de face de l'appareil uro-génital masculin après chirurgie après activation du dispositif hémostatique selon la figure 35,
- la figure 37 est une vue de face de l'appareil uro-génital masculin après chirurgie après activation du dispositif hémostatique selon la figure 35, et après instillation de l'agent thérapeutique dans la cavité prostatique.

Quel que soit le mode de réalisation décrit, le dispositif hémostatique selon l'invention, comprend au moins un cathéter urétral 11 avec un premier canal 25b, un premier ballonnet 24a et un dispositif de blocage 12.

Quel que soit le mode de réalisation du dispositif hémostatique10, le cathéter 11 comprend un corps comportant une extrémité distale 22c, une extrémité proximale 22a opposée à l'extrémité distale 22c. L'extrémité distale 22c présente préférentiellement une forme ronde lisse fermée, ce qui permet une insertion non traumatique du cathéter 11 dans l'urètre. La souplesse du cathéter 11 est voisine de celle d'une sonde de Foley classique.

Quel que soit le mode de réalisation du dispositif hémostatique 10, le cathéter 11 comprend en outre, un premier ballonnet 24a gonflable. Le premier ballonnet 24a étant destiné à être placé dans la vessie contre le col de la vessie et étant configuré pour exercer une pression à l'état gonflé sur la cavité prostatique de manière à obturer ladite cavité prostatique comme un couvercle et à réduire son volume et à occlure les vaisseaux de la paroi de la cavité prostatique. Le diamètre du premier ballonnet 24a est de l'ordre de 50 mm. Le premier ballonnet 24a comprend une forme invariable et des dimensions invariables après gonflage. Après gonflage, le premier ballonnet 24a a de préférence la forme cylindrique présentant un diamètre et une hauteur standard. La forme et les dimensions sont invariables après gonflage. La rigidité du premier ballonnet 24a gonflé exige : un gonflage à une pression de l'ordre de plus de 2 Bars avec un volume standard de liquide. La résistance de la paroi 21 est liée au choix du matériau composant la paroi 21 et à l'épaisseur de la paroi. Comme précisé plus haut, le matériau est préférentiellement le nylon. Les tissus en contact avec le ballonnet doivent se conformer à la forme du premier ballonnet 24a. Le premier ballonnet 24a ne doit pas se déformer ni se conformer à la forme des tissus.

Quel que soit le mode de réalisation du dispositif hémostatique 10, le premier ballonnet 24a présente un revêtement hydrophile, au moins sur la moitié inférieure proximale du ballonnet, ce qui permet une fermeture étanche de l'orifice supérieur de la cavité prostatique 120. Le revêtement hydrophile empêche les fuites de l'agent thérapeutique 50 instillé à l'intérieur de la cavité 120. Le cathéter urétral 11 comprend également un premier canal 25b relié fluidiquement au premier ballonnet 24a et configuré pour permettre le gonflage du premier ballonnet 24a. Le premier canal 25b est équipé d'une valve 26a pour gonfler le premier ballonnet 24a,

Quel que soit le mode de réalisation du dispositif hémostatique 10 décrit, le dispositif de blocage 12 est configuré pour fixer la position longitudinale du cathéter en tension par rapport au dispositif de blocage 12 et par rapport aux éléments anatomiques fixes du pelvis tels que le cadre osseux, le diaphragme périnéal et le sphincter urétral. Le dispositif de blocage 12 comprend au moins un organe de blocage.

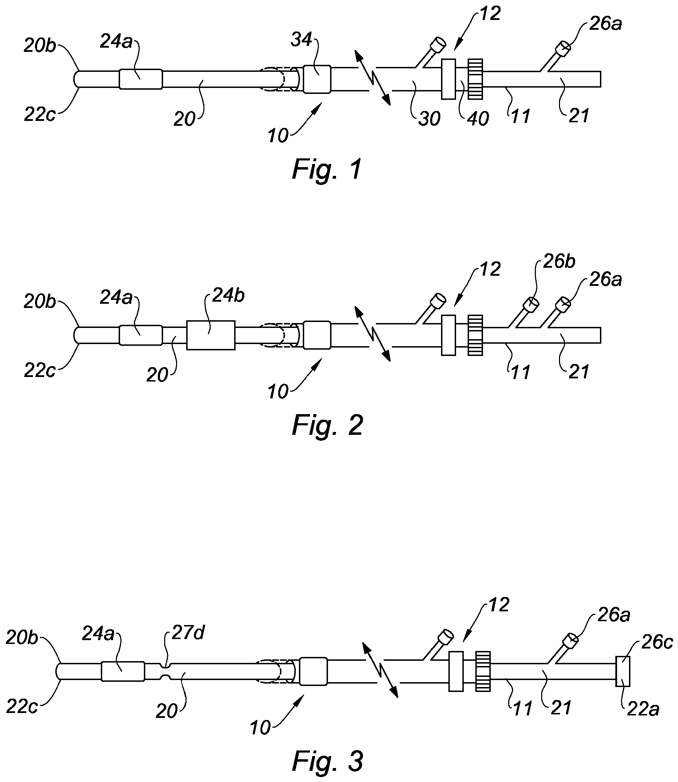

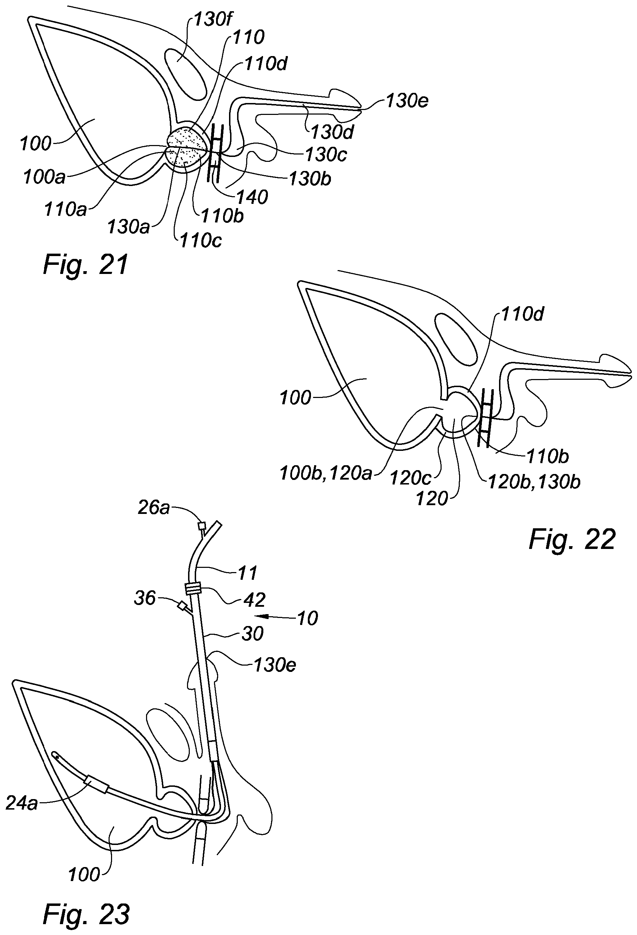

Les figures 21 à 26 décrivent schématiquement la mise en place et l'activation du dispositif hémostatique 10 quel que soit le mode de réalisation avec un dispositif de blocage selon le premier exemple. Pour une question de simplicité des figures, le dispositif hémostatique 10 illustré, sera le dispositif hémostatique 10 selon le premier mode de réalisation représenté figure 1 mais il pourrait s'agir des autres dispositifs hémostatiques sans que cela ne change la mise en place et l'activation.

Dans toute la demande, on entendra par « état non activé » du dispositif hémostatique 10, un état dans lequel, le dispositif hémostatique 10 n'exerce pas de pression et dans lequel, le ou les ballonnets sont en état dégonflé ou gonflé. Dans toute la demande, on entendra par « état activé » du dispositif hémostatique 10, un état dans lequel, le dispositif hémostatique 10 exerce une pression et dans lequel, le ou les ballonnets sont en état gonflé.

La figure 21 présente les structures anatomiques avant chirurgie suivantes : la vessie 100, le col de la vessie 100a, la prostate 110, la base de la prostate 110a, l'apex de la prostate 110b, le sphincter strié urinaire 140, l'urètre prostatique 130a, l'urètre membraneux 130b, l'urètre bulbaire 130c, l'urètre pénien 130d, le méat urétral 130e et l'os du pubis 130f. L'urètre masculin comprend quatre segments. L'urètre masculin débute au niveau de l'orifice de la vessie 100, d'où va s'écouler l'urine, il traverse la prostate 110 (segment prostatique 130a) de sa base 110a à son apex 110b, s'engage dans le sphincter strié 140 (segment membraneux 130b), traverse le périnée (segment bulbaire ou périnéal 130c, segment fixe de l'urètre), et enfin le pénis (segment pénien 130d, segment mobile de l'urètre) pour se terminer à l'extrémité du pénis par un orifice le méat urétral 130e.

Le diamètre de l'urètre varie considérablement selon le segment considéré. Le diamètre est étroit au niveau du col de la vessie 100a, au niveau du sphincter strié 140 et du méat urétral 130e. Le plus grand diamètre est observé au niveau du segment bulbaire 130c avec un diamètre deux à quatre fois supérieur à celui du segment pénien 130d. L'hypertrophie bénigne de la prostate se développe à partir des glandes péri-urétrales, situées dans la zone de transition 110c située au centre de la prostate et autour du segment prostatique 130a de l'urètre. La croissance de la zone de transition 110c avec la progression de l'âge entraîne la compression du segment prostatique 130a de l'urètre et un obstacle à la vidange de la vessie 100. Un plan anatomique délimite la zone de transition hypertrophiée 110c et la partie périphérique de la glande prostatique 110, appelée zone périphérique 110d. Ce plan est la limite de sécurité à ne pas franchir et à préserver au cours de la chirurgie endoscopique de l'hypertrophie bénigne de la prostate. La zone périphérique doit être préservée, car elle est riche en vaisseaux et est entourée sur son versant extérieur par un réseau vasculaire veineux développé à son contact.

En figure 22, après chirurgie, est illustrée une vue de la vessie 100, et de la cavité prostatique 120 après l'ablation de l'hypertrophie bénigne de la prostate, c'est-à-dire de la zone de transition 110c absente de la figure 22.

La chirurgie de l'hypertrophie bénigne de la prostate consiste à découper et enlever l'urètre prostatique 130a et la zone de transition hypertrophiée 110c, entourant et comprimant l'urètre prostatique 130a et à laisser en place la zone périphérique 110d sans la perforer. La zone périphérique 110d représente la paroi de la cavité 120c créée à l'intérieur de la prostate 110 par l'opération. Le segment prostatique 130a de l'urètre est supprimé avec la suppression de la zone de transition hypertrophiée 110c. La cicatrisation du segment prostatique 130a de l'urètre enlevé lors de l'opération et de la cavité formée va se faire à partir de la muqueuse urothéliale qui est le revêtement superficiel de l'ensemble des voies urinaires. Une nouvelle couche de muqueuse urothéliale se développe en haut de la cavité 120 à partir de l'orifice de la vessie 100b qui est devenu l'orifice supérieur 120a de la cavité prostatique 120, et en bas à partir de l'orifice inférieur 120b de la cavité 120 qui correspond à la section de l'urètre prostatique 130a à l'apex de la prostate 110b au dessus du sphincter strié 140. Au niveau de la paroi de la cavité, la muqueuse pousse à partir de la tranche de section des canaux de la zone périphérique 110d de la prostate 110. La cavité 120 est progressivement tapissée par la nouvelle couche urothéliale en 3 mois. L'excellente circulation sanguine au niveau de la muqueuse de l'orifice de la vessie 100b en haut, de l'orifice de l'urètre 120b en bas ainsi qu'au niveau de la paroi prostatique 120c favorise la cicatrisation de la plaie créée par la chirurgie à l'intérieur de la prostate 110.

L'intégrité de la zone périphérique 110d doit être préservée pendant la chirurgie endoscopique. Toute perforation de la zone périphérique 110d expose à la blessure des vaisseaux sanguins entourant la prostate 110 latéralement, en bas à la blessure du sphincter strié 140, en arrière du rectum et latéralement à la blessure des lames neuro-vasculaires dont dépendent la continence urinaire et l'érection (les vaisseaux sanguins, le rectum, les lames vasculo-nerveuses ne sont pas représentés sur la figure). En conséquence une hémorragie, une incontinence urinaire, une fistule recto-urétrale, une impuissance sexuelle d'origine neurologique peuvent être observés après cette chirurgie.

Comme illustré en figure 23, le pénis est étiré verticalement et le dispositif 10 est inséré dans l'urètre. Le cathéter 11 est introduit en premier, puis le tube de blocage 30 préalablement monté sur le cathéter 11. Le cathéter 11 est poussé dans la vessie à travers le tube de blocage, jusqu'à ce que le premier ballonnet 24a soit correctement positionné à l'intérieur de la vessie 100.

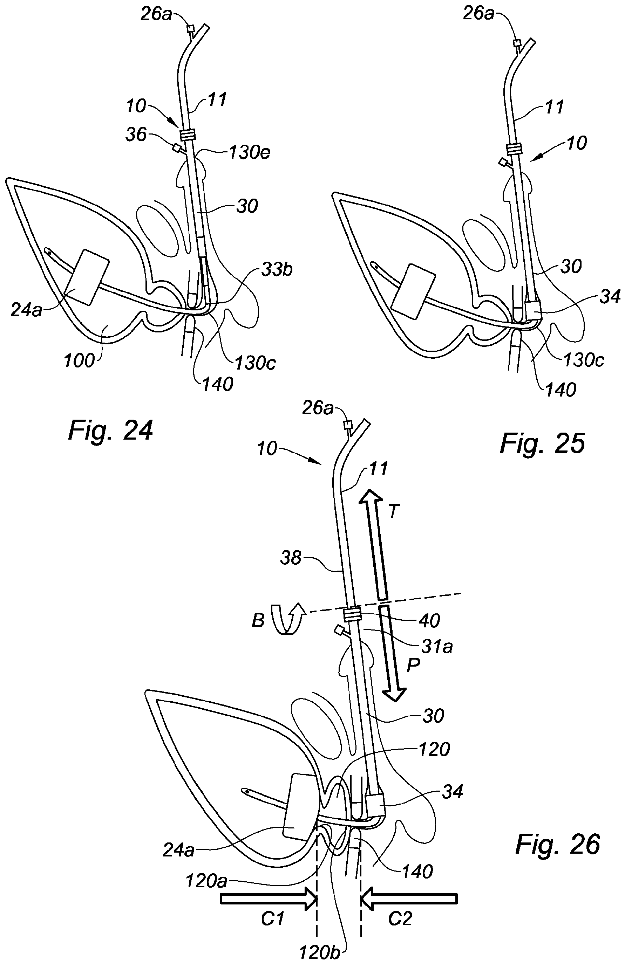

Comme illustré en figure 24, le premier ballonnet 24a du cathéter 11 est alors gonflé à l'intérieur de la vessie 100. Tout en étirant le pénis verticalement, l'opérateur pousse le tube de blocage 30 à travers le méat urétral 130e, jusqu'à ce que l'extrémité distale 33b du tube de blocage 30 bute contre la paroi postérieure de l'urètre bulbaire 130c, c'est-à-dire sous le sphincter strié 140.

Comme illustré en figure 25, le troisième ballonnet 34 du tube de blocage 30 est gonflé dans l'urètre bulbaire 130c sous le sphincter strié 140. Le dispositif 10 se trouve dans un état non activé avec les ballonnets gonflés.

Comme illustré en figure 26, le cathéter 11 est tiré d'une main par l'opérateur, tandis que de l'autre main il maintient la position du troisième ballonnet 34 du tube rigide 30 au contact et en dessous du sphincter strié 140. Une fois que l'opérateur sent une résistance suffisante à la traction du cathéter 11, la position relative du cathéter 11 et du tube 30 est fixée par l'activation de l'organe de blocage 40 situé sur l'extrémité proximale 31a du tube de blocage 30. Ce blocage en tension de la position relative du cathéter 11 et du tube de blocage 30 permet de maintenir la compression de la cavité prostatique 120 par les deux ballonnets, appuyant de chaque côté de la cavité. Le premier ballonnet 24a appuie au niveau de son orifice supérieur 120a et le troisième ballonnet 34 appuie au niveau de son orifice inférieur 120b par l'intermédiaire du sphincter strié 140. A ce stade le dispositif hémostatique 10 est dans un état activé.

Les flèches situées de part et d'autre d'une ligne pointillée passant au niveau de l'organe de blocage 40, représentent les forces exercées par l'opérateur : une force de traction T sur le cathéter 11 avec une main et à l'opposé une force de poussée P avec l'autre main pour maintenir le troisième ballonnet 34 du tube de blocage 30 au-dessous du sphincter strié 140. La flèche courbe B représente la force utilisée pour l'activation de l'organe de blocage 40, une fois que le cathéter 11 et le tube de blocage 30 sont correctement positionnés et que la cavité prostatique 120 est non seulement comprimée mais également fermée de chaque côté.

La flèche C1 pointant sur la ligne pointillée passant par l'orifice supérieur 120a de la cavité prostatique 120 représente la force transmise au premier ballonnet 24a et exercée par le premier ballonnet 24a pour comprimer et fermer l'orifice supérieur 120a de la cavité prostatique.

La flèche C2 pointant sur la ligne pointillée passant au contact du sphincter strié 140 représente la force transmise par le troisième ballonnet 34 et exercée par le troisième ballonnet 34 contre la face inférieure du sphincter strié 140, pour comprimer par l'intermédiaire du sphincter 140 l'orifice inférieur 120b de la cavité prostatique 120.

Bien entendu, et comme dit précédemment en introduction des figures 21 à 26, l'insertion du dispositif hémostatique et le concept général d'activation reste le même quel que soit le mode de réalisation du dispositif hémostatique, selon les variantes des différents modes, le gonflement du deuxième ballonnet et/ou l'instillation d'un agent thérapeutique ou le gonflement du troisième ballonnet sera additionnel, en fonction du cathéter et du dispositif blocage caractérisant le mode de réalisation du dispositif hémostatique utilisé.

Le premier mode de réalisation du dispositif hémostatique 10 selon l'invention va maintenant être décrit en référence à la figure 1.

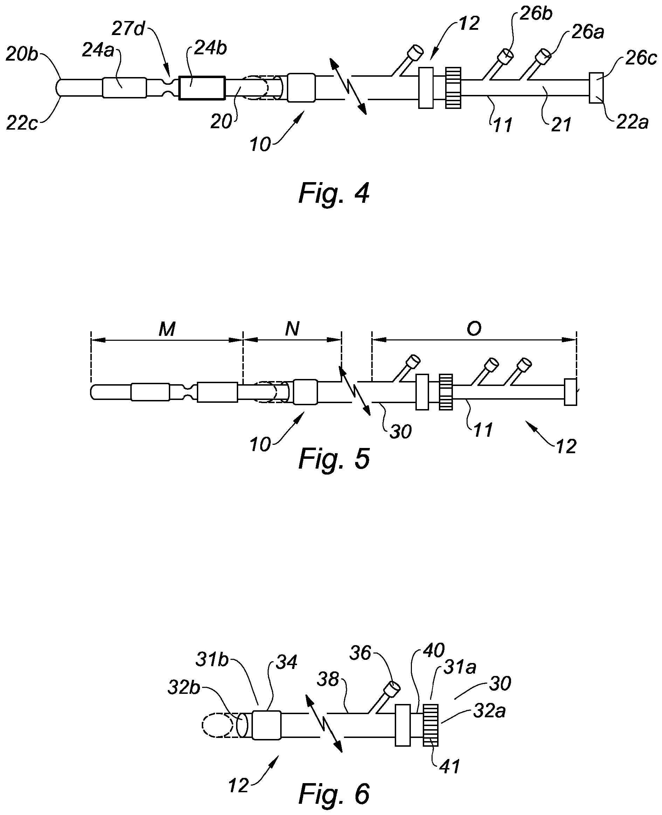

Dans le premier mode de réalisation du dispositif hémostatique, ledit dispositif hémostatique 10 comprend un cathéter 11 comprenant un corps 20 équipé d'un dispositif de blocage 12 de position en tension. Le dispositif de blocage 12 est emmanché sur le cathéter 11. Le cathéter 11 comprend en outre un premier ballonnet 24a à une portion 20b d'une première extrémité distale 22c du cathéter 11. Le premier ballonnet 24a est destiné à être placé dans la vessie 100 contre le col de la vessie 100a (figure 26) et étant configuré pour exercer une pression déterminée à l'état gonflé sur la cavité prostatique 120 de manière à obturer et isoler la cavité prostatique 120, à réduire le volume de la cavité prostatique 120 et à occlure les vaisseaux de la paroi de la cavité prostatique 120. Le cathéter 11 comprend un premier canal 25b (non représenté sur la figure 1) en connexion fluidique avec le premier ballonnet 24a. Le premier canal 25b permet le gonflage du premier ballonnet 24a par la valve 26a. A côté du canal 25b, le reste du corps du cathéter 11 est plein sur toute sa longueur. Le dispositif de blocage 12 du dispositif hémostatique 10 selon un premier exemple de réalisation sera décrit plus loin dans la description en référence à la figure 6. Succinctement, le dispositif de blocage 12 selon le premier exemple est en contact direct avec le cathéter; le dispositif de blocage 12 consiste en un tube de blocage 30 pourvu entre autres d'un organe de blocage 40 et d'un troisième ballonnet 34.

Dans une première variante du premier mode de réalisation illustrée en figure 2, le dispositif hémostatique 10 comprend un deuxième ballonnet 24b situé en dessous du premier ballonnet distal 24a. Dans cette première variante, le cathéter 11 comprend un deuxième canal 25c. Le deuxième canal 25c (non représenté sur la figure 2) est relié fluidiquement au deuxième ballonnet 24b et permet le gonflage dudit deuxième ballonnet 24b par une valve 26b. A la différence du premier ballonnet 24a décrit plus haut, le deuxième ballonnet 24b est configuré pour s'adapter à la forme des tissus et particulièrement à la forme irrégulière de la cavité prostatique dans laquelle il est gonflé.

Ainsi le premier ballonnet 24a et le deuxième ballonnet 24b contribuent mécaniquement à l'hémostase ; dans un premier temps le premier ballonnet 24a par compression exercée de l'extérieur de la cavité, puis dans un deuxième temps le deuxième ballonnet 24b par compression exercée depuis l'intérieur de la cavité. La paroi de la cavité est comprimée de chaque côté, de l'extérieur et de l'intérieur.

Le deuxième ballonnet 24b facilite aussi la propagation de l'agent thérapeutique 50 et son contact avec la face interne de la paroi 120c de la cavité prostatique 120 comme décrit plus loin.

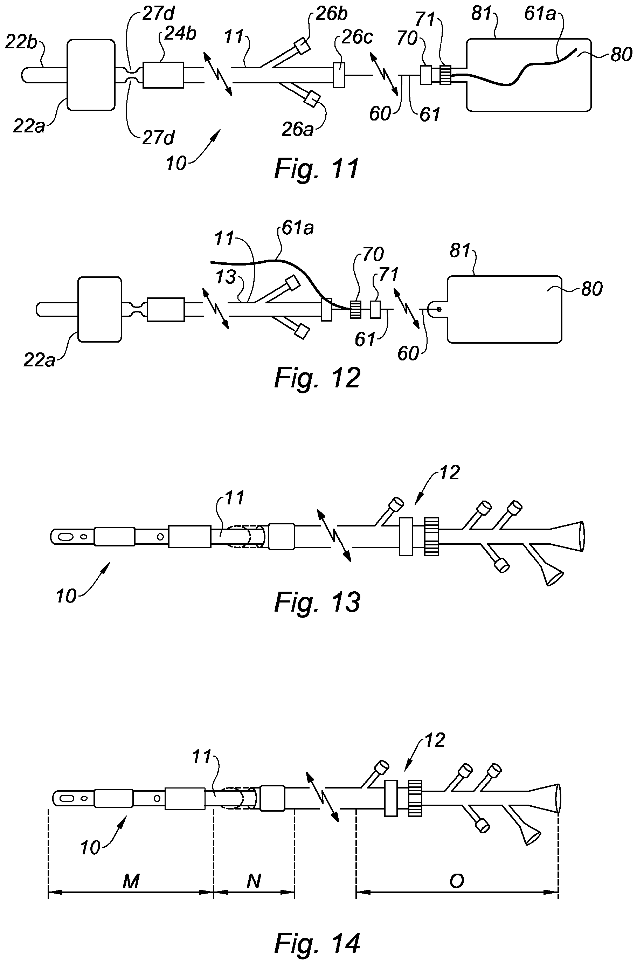

Dans une deuxième variante non représentée du premier mode de réalisation du dispositif hémostatique 10, le dispositif de blocage selon un deuxième exemple de réalisation est dépourvu du tube 30 et du troisième ballonnet 34. Ce deuxième exemple de réalisation du dispositif de blocage sera décrit plus loin dans la description en référence à la figure 11. Les autres caractéristiques du premier mode de réalisation et de la première variante du premier mode de réalisation sont les mêmes et sont valables pour cette deuxième variante du premier mode de réalisation.

Les caractéristiques du premier mode de réalisation et de ces variantes se retrouvent dans le deuxième mode de réalisation du dispositif hémostatique 10 selon l'invention et seront plus détaillées par la suite notamment dans la description en référence à la figure 3 illustrant le deuxième mode de réalisation du dispositif hémostatique et aux figures 4, 5, 7A à 7F 8, 9 et 10 illustrant la première variante du deuxième mode de réalisation.

Le deuxième mode de réalisation du dispositif hémostatique 10 selon l'invention, illustré en figure 3, diffère du premier mode de réalisation en ce que le dispositif hémostatique 10 comprend un troisième canal 25e (non représenté sur la figure 3) configuré pour acheminer depuis un orifice d'entrée ou une valve 26c un agent thérapeutique 50. Le troisième canal 25e comprend en outre un orifice de sortie 27d destiné à déboucher à l'intérieur de la cavité prostatique en état d'utilisation. Le reste des caractéristiques du deuxième mode de réalisation sont les mêmes que dans le premier mode de réalisation.

Dans le deuxième mode de réalisation du dispositif hémostatique 10, le diamètre du troisième canal 25e est au moins quatre fois supérieur au diamètre d'un canal pour le gonflage d'un ballonnet. Ce diamètre est nécessaire pour faciliter l'instillation du gel contenant l'agent thérapeutique 50. La taille de l'orifice de sortie 27d est adaptée au diamètre du canal 25e. Un deuxième orifice de sortie 27d, symétrique du premier par rapport à l'axe longitudinal du cathéter, facilite la sortie et la répartition symétrique de l'agent thérapeutique à l'intérieur de la cavité.

Quel que soit le mode de réalisation du dispositif hémostatique 10 comprenant une option d'instillation d'un agent thérapeutique (deuxième mode de réalisation et troisième mode de réalisation), l'agent thérapeutique 50 instillé dans la cavité 120 appartient à l'une des classes suivantes de produits qui peuvent être utilisés seuls ou en combinaison et sans limitation aux classes ou produits suivants:
- les agents alpha-mimétiques : ce sont des drogues vaso-constrictives qui réduisent le diamètre des vaisseaux et par conséquent réduisent la perfusion sanguine des organes.
- les agents coagulants par exemple la fibrine, la thrombine renforcent l'hémostase naturelle par un effet direct sur l'hémostase.
- les agents embolisants soit sous forme liquide, soit sous forme de particules ou microsphères peuvent être proposés, par exemple le Gelfoam (Pfizer). Cet agent composé de particules de gélatine est régulièrement utilisé pour l'embolisation des veines spermatiques dans le traitement de la varicocèle chez l'homme.
- les agents sclérosants peuvent être proposés mais à condition de rendre acceptable la réaction inflammatoire qu'ils provoquent.
- les agents de remplissage ou de comblement : ces agents ne participent pas à la cascade de la coagulation naturelle. Ils ont une action directe non spécifique purement mécanique sur l'hémostase. Le remplissage d'une cavité avec un agent de comblement réduit la taille de l'espace libre à l'intérieur de la cavité. L'agent de comblement vient occuper l'espace qui en l'absence d'agent de comblement serait occupé par la formation de caillots. L'agent de comblement contribue à l'hémostase simplement par son effet de tamponnade mécanique appliquée contre la paroi 120c de la cavité 120. Il présente l'avantage de réduire la taille du caillot naturel et de ne pas libérer de facteurs fibrinolytiques. L'agent de comblement peut se dissoudre spontanément et être éliminé avec l'urine. Les agents de comblement suivants peuvent être proposés : le gel Legoo est proposé par Pluromed, une division de la société Sanofi-Aventis; le polyvinyl-pyrolidone est commercialisé par la société BASF ; un gel d'acide hyaluronique peut être proposé.
- une nouvelle classe, celle des peptides auto-agrégeant combine avantageusement un effet coagulant à l'effet de comblement. L'ensemble de peptides auto-agrégeant est synthétique et sensible au PH environnant. Le contact avec le PH sanguin provoque l'agrégation des peptides. La densité du gel augmente très nettement et provoque l'occlusion des vaisseaux. Cette classe d'agent permet l'hémostase à l'échelle macroscopique, à l'échelle microscopique et même nanométrique. Cette classe d'agent apparaît comme la plus appropriée pour l'utilisation du dispositif. Le Purastat® ou TDM 621 de la société 3D-Matrix est un exemple de cette nouvelle classe thérapeutique.

Avantageusement, l'agent thérapeutique se présente sous la forme d'un gel dont la viscosité est supérieure à celle d'une solution d'irrigation classiquement utilisée.

Ainsi, l'hémostase de la cavité prostatique est assurée par une double action : la combinaison d'une action mécanique et d'une action biologique et/ou biochimique. L'action mécanique est assurée par deux ballonnets, le premier ballonnet 24a et le deuxième ballonnet 24b situés respectivement à l'extérieur et à l'intérieur de la cavité ; l'action biologique est assurée par les facteurs de coagulation naturelle apportés par le sang dans la cavité ; l'action biochimique est assurée par l'instillation d'un agent thérapeutique dont l'effet est facilité par le deuxième ballonnet 24b gonflé à l'intérieur de la cavité.

Dans une première variante du deuxième mode de réalisation illustrée en figure 4, le dispositif hémostatique 10 comprend un deuxième ballonnet 24b illustré sur la figure 4. Dans cette première variante, le cathéter 11 comprend un deuxième canal 25c non représenté en figure 4. Le deuxième canal 25c est relié fluidiquement au deuxième ballonnet 24b et permet le gonflage dudit deuxième ballonnet 24b par une valve 26b.

Le deuxième ballonnet 24b est configuré pour comprimer de l'intérieur la paroi de la cavité prostatique comme décrit ci-dessus, et pour faciliter la propagation de l'agent thérapeutique 50 et son contact avec la face interne de la paroi 120c de la cavité prostatique 120.

Les caractéristiques de la première variante du deuxième mode de réalisation seront plus détaillées par la suite notamment en référence aux figures 4 à 7F et 8 à 10.

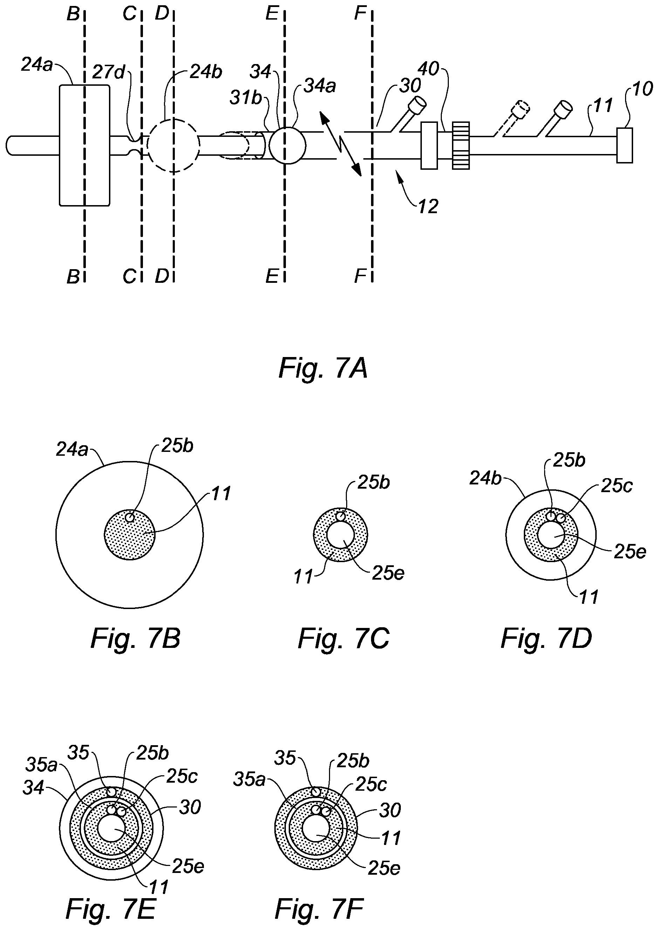

Les figures 7B à 7F montrent le nombre des canaux et la position des canaux selon les plans de section BB à FF représentés sur la figure 7A. Ainsi, sont représentés, le premier canal 25b du gonflage du premier ballonnet 24a, le deuxième canal 25c de gonflage du deuxième ballonnet 24b, le troisième canal 25e d'instillation de l'agent thérapeutique 50. Ces trois canaux permettent la différenciation du deuxième mode de réalisation première variante des autres modes de réalisation et variantes.

Chaque canal chemine dans la paroi du cathéter. Le canal central 35a du tube de blocage 30 permet le passage du cathéter 11. Un quatrième canal 35 de gonflage du troisième ballonnet 34 chemine dans la paroi du tube de blocage 30.

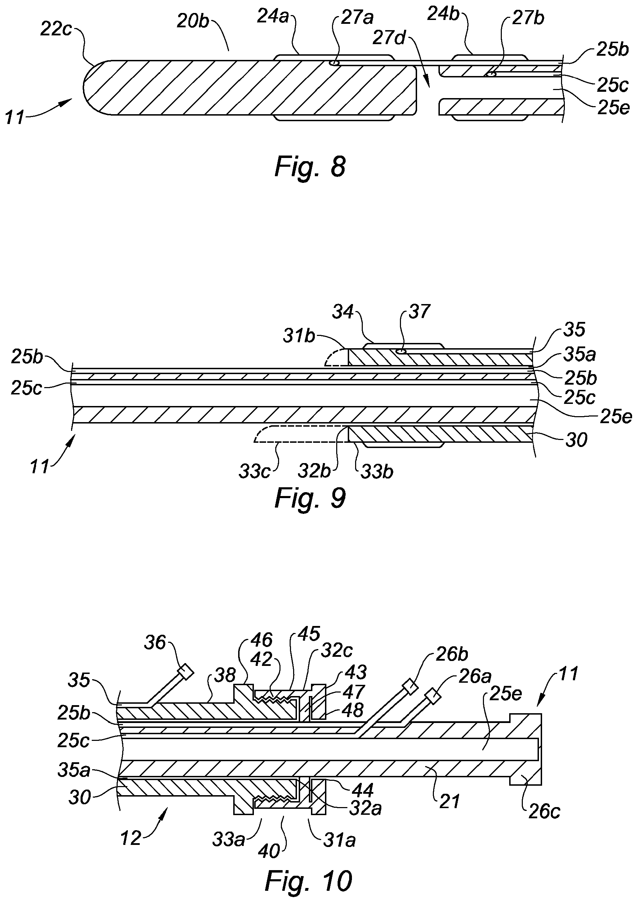

Les figures 8 à 10, montrent le nombre, le trajet des canaux le long du cathéter 11 et du tube de blocage 30, ainsi que les positions respectives de ces canaux selon le segment M, N, et O du dispositif tels que représentés en figure 5.

Dans une deuxième variante, illustrée figure 11, du deuxième mode de réalisation du dispositif hémostatique 10, le dispositif de blocage selon le 2°exemple de réalisation est dépourvu du tube 30 et du troisième ballonnet 34. Les autres caractéristiques de la première variante du deuxième mode de réalisation sont les mêmes et sont valables pour cette deuxième variante du deuxième mode de réalisation.

Comme décrit précédemment, le dispositif hémostatique 10 comprend un dispositif de blocage 12 selon un premier exemple de réalisation illustré à la figure 6. Le dispositif de blocage 12 selon le premier exemple de réalisation comprend un tube de blocage 30 coopérant avec un organe de blocage 40. Les détails sur l'organe de blocage 40 et sur la position relative du tube de blocage 30 et du cathéter 11 sont représentés sur les figures 9 et 10.

Selon le premier exemple de réalisation, le tube de blocage 30 comprend un tube 38. Le tube 38 est préférentiellement semi-rigide. Le tube de blocage 30 comprend également un troisième ballonnet 34 gonflable, et dont le gonflement est contrôlé par un manomètre. Le troisième ballonnet 34 présente, après gonflage, une forme préférentiellement sphérique. En outre, l'élasticité de la paroi du troisième ballonnet 34 et sa pression de gonflage sont proches de celles du ballonnet d'une sonde d'intubation trachéale. Après gonflage, le diamètre du ballonnet est de l'ordre de 2 fois le diamètre externe du cathéter. Il est important qu'après gonflage, le troisième ballonnet 34 puisse glisser librement à l'intérieur de la portion bulbaire 130c de l'urètre, lorsque le dispositif hémostatique 10 est dans un état non activé. La pression de gonflage du troisième ballonnet 34 est basse c'est-à-dire qu'elle ne doit pas dépasser la pression artérielle systolique, pour éviter toute compression ischémique de la muqueuse de la portion bulbaire de l'urètre 130c. Le troisième ballonnet 34 est situé à la partie distale 31b du tube de blocage 30. Une valve 36 située à son extrémité proximale 31a permet le gonflage dudit troisième ballonnet 34.

Selon le premier exemple de réalisation, le tube de blocage 30 comprend un quatrième canal 35 cheminant dans la paroi du tube de blocage 30 pour permettre le gonflage du troisième ballonnet 34 situé à l'extrémité distale 31b du tube de blocage 30 comme illustré figures 9 et 10.

Selon le premier exemple de réalisation, le tube de blocage 30 présente une longueur et un diamètre unique et adaptés à ceux du cathéter 11. La longueur du tube de blocage 30 est environ la moitié de celle du cathéter 11. Avantageusement, le tube de blocage 30 est emmanché par coulissement sur le cathéter 11. Le tube de blocage 30 comprend un orifice proximal 32a et un orifice distal 32b qui permettent l'insertion du cathéter 11 à l'intérieur dudit tube de blocage 30.

Selon le premier exemple de réalisation, le tube de blocage 30 comprend un canal central 35a avec un diamètre adapté au diamètre extérieur du cathéter 11, de façon à ce que le tube de blocage 30 glisse le long du cathéter 11 et de façon étanche sans passage de sang ou de l'agent thérapeutique 50 entre le cathéter 11 et le tube de blocage 30. L'orifice proximal 32a et l'orifice distal 32b du tube de blocage 30 sont situés dans un plan perpendiculaire à l'axe du tube de blocage 30. L'extrémité proximale 31a du tube de blocage 30 est équipée d'un organe de blocage 40. L'orifice 32b à l'extrémité distale 33b du tube de blocage 30 se situe juste au-dessus du troisième ballonnet 34 du tube de blocage 30. L'extrémité distale 33b du tube de blocage 30 vient se positionner juste en dessous du sphincter strié 140 dans l'urètre bulbaire 130c.

Selon une variante de l'extrémité distale 33b du tube de blocage 30, une forme en biseau 33c représentée par une ligne en pointillés sur les figures 1 à 6 et figure 9 notamment, permet de faciliter l'insertion du tube de blocage 30 dans le méat urétral 130e. Dans cette variante, le biseau 33c traverse le sphincter strié 140 et se termine à l'intérieur de la cavité prostatique 120. La paroi de l'extrémité en biseau 33c doit être aussi souple que la paroi 21 du cathéter 11. La souplesse de l'extrémité en biseau 33c doit contraster avec la rigidité de la paroi du tube de blocage 30, de sorte que l'extrémité distale 33b de la partie semi-rigide du tube de blocage 30 vienne buter contre la paroi postérieure de l'urètre bulbaire 130c (se référer plus loin aux figures 24 à 29). La sensation de butée doit ainsi être facilement perçue par l'opérateur, lors de l'insertion et positionnement du troisième ballonnet 34 du tube de blocage 30 contre et en dessous du sphincter strié 140.

Selon le premier exemple de réalisation, l'organe de blocage 40 est configuré pour fixer la position longitudinale relative du cathéter 11 en tension et du tube de blocage 30. L'organe de blocage 40 est situé sur le tube de blocage 30. L'organe de blocage 40 est en contact direct avec le cathéter 11.

Comme illustré en figure 10, l'organe de blocage 40 selon le premier exemple de réalisation, comprend un boulon 41, situé à l'extrémité proximale 31a du tube de blocage 30, ledit boulon 41 comprend une partie mâle et son homologue femelle. La partie mâle correspond à la surface extérieure filetée de l'extrémité proximale 31a du tube de blocage 30 sur une longueur de l'ordre de 5 mm. Partant de l'orifice 32a du tube de blocage 30, le filet 45 se termine contre une butée circulaire 46 dépassant de 2 mm au moins la surface externe de la gaine 38 du tube de blocage 30. La partie femelle est un écrou 42 recouvert d'un capuchon rigide 43 sur sa face proximale. Le capuchon rigide 43 est fixé sur l'écrou 42 et est percé par un orifice central 44 de diamètre correspondant au diamètre extérieur du cathéter 11. Un joint en silicone 47 est situé entre le bord de l'orifice 32c situé à l'extrémité proximale 33a du tube de blocage 30 et l'écrou 42. Le joint 47 a un orifice central 48 de diamètre 22 CH pour permettre l'insertion du cathéter 11 à l'intérieur du joint 47 et un diamètre extérieur permettant sa mise en place à l'intérieur de l'écrou 42 et au contact de la face interne du capuchon 43 de l'écrou 42. Le serrage de l'écrou 42 situé à l'extrémité proximale 31a du tube de blocage 30 permet la compression du joint 47 entre le bord de l'orifice proximal 32c du tube de blocage 30 et la face interne du capuchon 43 de l'écrou 42. La compression du joint 47 entraîne l'expansion du joint 47, d'un côté contre la face externe compressible du cathéter 11, et à l'opposé contre le versant interne rigide de l'écrou 42.

Le serrage puis le blocage de l'écrou 42 contre la butée circulaire 46 du tube de blocage 30 comprime le joint 47 et le cathéter 11 selon une force de blocage standardisée et reproductible d'un patient à l'autre. Les caractéristiques standardisées du cathéter 11 et celles du joint 47 contribuent à la standardisation et reproductibilité de la force de blocage. Le serrage puis le blocage de l'écrou 42 fixe la position relative du cathéter 11 et du tube de blocage 30. La fixation de la position relative du cathéter 11 et du tube de blocage 30 est effectuée après gonflage du premier ballonnet 24a et du troisième ballonnet 34, et ensuite après compression de la cavité prostatique 120 par le premier ballonnet 24a et le troisième ballonnet 34. La compression de la cavité prostatique 120 est obtenue en tirant le cathéter 11 et en maintenant l'extrémité distale 33b du tube de blocage 30 contre le versant postérieur de l'urètre bulbaire 130c et en dessous du sphincter strié 140. Après blocage de l'écrou 42, toute tension excessive entraîne le glissement du cathéter 11 à l'intérieur du joint 47 de l'organe de blocage 40 jusqu'au retour à la tension standard qui bloque la position du cathéter 11 en tension. Ainsi, la cavité prostatique 120 est soumise à une force de compression longitudinale standardisée et reproductible.

Comme illustré aux figures 8, 9 et 10, le corps du cathéter 11 comprend une valve 26b et un deuxième canal 25c comprenant un orifice 27b; la valve, le deuxième canal 25c et l'orifice 27b étant reliés fluidiquement les uns aux autres et au deuxième ballonnet 24b. La liaison fluidique entre la valve, le deuxième canal 25c et l'orifice 27b permet le gonflage du deuxième ballonnet 24b. Le canal 25c chemine dans la paroi 21 du cathéter 11.

Après gonflage le deuxième ballonnet 24b présente une forme préférentiellement sphérique donc différente de celle du premier ballonnet 24a qui est préférentiellement cylindrique. Après gonflage, la paroi du deuxième ballonnet 24b vient ou non se plaquer contre la paroi 120c de la cavité prostatique 120.

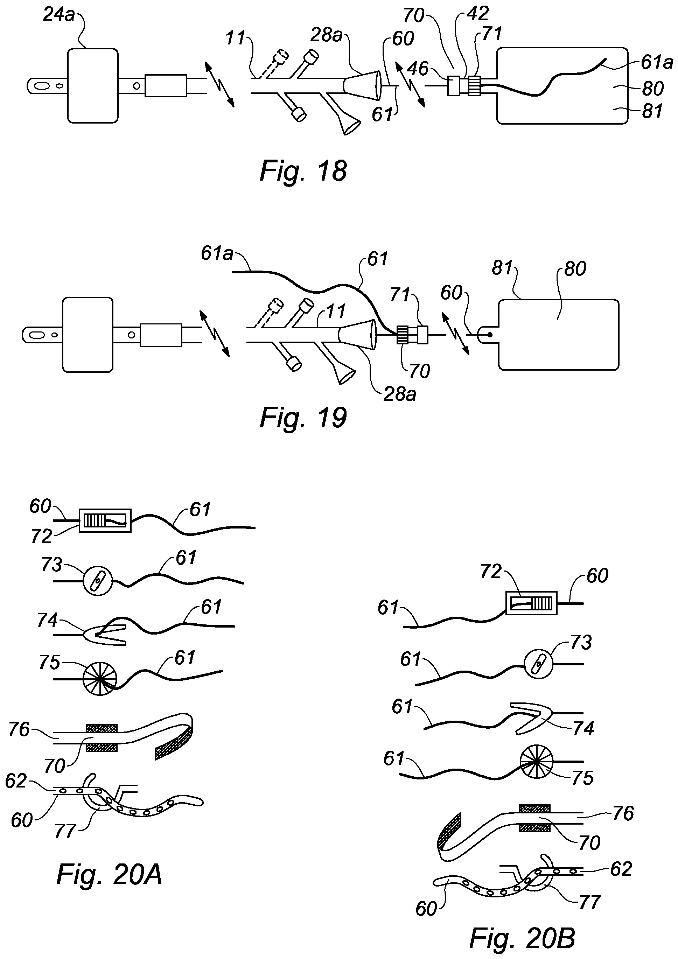

En référence aux figures 11 et 20A, le dispositif hémostatique 10 selon la première variante du deuxième mode de réalisation comprend un dispositif de blocage 12 selon un deuxième exemple de réalisation. Le dispositif de blocage 12 selon le deuxième exemple de réalisation diffère du dispositif de blocage 12 selon le premier exemple de réalisation (illustré en figure 6) en ce que le tube de blocage 30 est supprimé. En conséquence, il n'y a pas plus de troisième ballonnet 34 dans l'urètre bulbaire 130c. L'organe de blocage 70 n'est plus positionné à l'intérieur du patient mais à l'extérieur du patient. L'organe de blocage est attaché à un moyen d'arrimage 80 fixé à la paroi de l'abdomen du patient. L'organe de blocage 70 n'est plus en contact direct avec le cathéter. L'organe de blocage 70 a un contact indirect avec le cathéter 11 par l'intermédiaire d'un moyen de raccordement 60. Comme illustré en figure 20A, le moyen de raccordement 60 est soit un fil 61, ou une tige souple 62, une bande 63, tout moyen doté d'une certaine souplesse.

Comme illustré en figure 11, le moyen de raccordement 60 a une extrémité attachée à la valve 26c d'instillation de l'agent thérapeutique du cathéter 11 et son extrémité opposée libre 61a est connectée à, ou coulisse à travers, ou est roulée à l'intérieur et est bloquée par l'organe de blocage 70. En effet, l'organe de blocage 70 est proposé selon plusieurs variantes non limitées à celles décrites ci-dessous dans les figures 20A et 20B.

Comme représenté en figure 11, l'organe de blocage 70 peut être un boulon 71 comparable à l'organe de blocage 40 du tube de blocage 30 du dispositif de blocage 12 selon le premier exemple de réalisation représenté en détails sur la figure 10. Cependant, le boulon 71 de l'organe de blocage du dispositif de blocage 12 selon le deuxième exemple de réalisation présente une différence majeure avec le boulon 41 de l'organe de blocage du dispositif de blocage 12 selon le premier exemple de réalisation pour ce qui concerne sa partie mâle :
- ses limites en longueur sont les suivantes (se reporter à la figure 10) l'origine de la partie filetée 45 (non représentée sur la figure 11) d'un côté et du côté opposé, la fin de la partie filetée 45 y compris la butée circulaire 46 contre laquelle l'écrou 42 sera bloqué. La partie mâle ne va pas au-delà du côté distal de la butée 46.
- les dimensions sont adaptées au type et aux dimensions du moyen de raccordement 60. Parmi les exemples de moyen de raccordement mais sans limitation à ces exemples, un fil 61, une tige souple 62 ou une bande 63 peuvent être proposés. Ces moyens de raccordement 60 ont comme caractéristique commune leur blocage par l'organe de blocage 70 selon une force de blocage standardisée et reproductible d'un patient à l'autre. Toute tension excessive du fil est automatiquement relâchée jusqu'au retour à la tension standard.

Comme illustré en figure 20A, l'organe de blocage 70 peut prendre plusieurs autres formes que celle d'un boulon 71.

L'organe de blocage 70 peut être une cage 72 avec une roue captive à l'intérieur de la cage 72. La roue comprime progressivement le moyen de raccordement 60, par exemple, un fil 61 contre le plancher de la cage 72. Lorsque la roue est en butée contre la paroi de la cage 72, le fil 61 est bloqué selon une force de compression standard. Toute tension excessive entraîne le glissement du fil sous la roue jusqu'au retour à la tension standard.

Alternativement, l'organe de blocage 70 peut être un winch 73 avec un fil 61 comme moyen de raccordement 60. Le winch bloque le fil selon une force de tension standard au-delà de laquelle toute tension excessive du fil est automatiquement relâchée jusqu'au retour à la tension standard.

Alternativement, l'organe de blocage 70 peut être une épingle 74 avec une fente permettant le blocage du fil 61. Toute tension excessive entraîne le glissement du fil à travers la fente jusqu'au retour à la tension standard.

Alternativement, l'organe de blocage 70 peut être une plaque circulaire 75 avec un orifice central et des fentes radiales à partir de l'orifice central. Toute tension excessive entraîne le glissement du fil à travers l'orifice central jusqu'au retour à la tension standard.

Alternativement, l'organe de blocage 70 peut être fait de bandes Velcro 76 avec une partie mâle et une partie femelle de dimensions standard. Toute tension excessive entraîne la déconnexion des deux bandes, jusqu'à ce que la tension standard soit atteinte et permette le maintien du montage.

Alternativement, l'organe de blocage 70 peut être un crochet 77 avec une élasticité réversible. Toute tension excessive exercée par la tige souple perforée 62 utilisée comme moyen de raccordement 60, aboutit à la déformation du crochet 77 et à la libération de la tige souple perforée 62. Le crochet déformé 77 récupère sa forme d'origine. Un nouveau raccordement est essayé avec une force plus légère ajustée à la résistance standard du crochet 77.

D'autres modes de réalisation peuvent être proposés, ils sont tous caractérisés par le fait qu'ils permettent de limiter et standardiser automatiquement la force de traction.

Selon le deuxième exemple de réalisation du dispositif de blocage en référence aux figures 11 et 20A, l'organe de blocage 70 est attaché à un moyen d'arrimage 80. Plusieurs variantes du moyen d'arrimage 80 sont proposées mais sans se limiter à, une plaque adhésive 81 ou une ceinture (non représentée sur les figures). La plaque adhésive 81 est collée sur le patient latéralement, sur le thorax en dessous du creux axillaire là où il n'y a pas de pilosité. La ceinture est serrée autour de l'abdomen du patient. L'activation de l'organe de blocage 70 selon le deuxième exemple de réalisation du dispositif de blocage représenté figure 11 est voisine de celle décrite lors de l'activation de l'organe de blocage 40 selon le premier exemple du dispositif de blocage dans les figures 23 à 26 illustrant l'insertion et l'activation du premier mode de réalisation du dispositif hémostatique 10 . Le cathéter 11 est mis en place dans l'urètre, le premier ballonnet 24a est gonflé dans la vessie.

Le moyen d'arrimage 80, par exemple une plaque adhésive 81 est collée au patient. L'extrémité libre du moyen de raccordement 60, un fil 61 par exemple est passé dans l'organe de blocage 70, par exemple un boulon 71 attaché au moyen d'arrimage 80. D'une main l'opérateur exerce une traction sur l'extrémité libre 61a du fil 61 dont l'extrémité opposée est fixée à l'extrémité proximale du cathéter. De l'autre main, l'opérateur maintient l'organe de blocage 70 en position stable. L'opérateur active l'organe de blocage 70 lorsque la tension exercée sur le cathéter 11 est jugée suffisante. La tension du cathéter 11 diminue et s'équilibre spontanément avec la résistance standard de l'organe de blocage 70. Le glissement du fil 61 dans l'organe de blocage, le boulon 71, s'arrête à un équilibre standardisé. La compression de la cavité est standardisée, calibrée, avec un réglage automatique pour obtenir la compression standard et l'exclusion de la cavité prostatique 120 ainsi que la compression et occlusion des vaisseaux de ladite cavité 120.

En variante de réalisation de la fixation de l'organe de blocage 70, ce dernier peut aussi être fixé au cathéter 11, au lieu d'être fixé au moyen d'arrimage 80 comme décrit ci-dessus en référence aux figures 11 et 20A. Selon cette variante illustrée aux figures 12 et 20B, la fixation de l'organe de blocage 70 se fait au niveau du cathéter. Une extrémité du moyen de raccordement 60 est fixée au moyen d'arrimage 80 et l'autre extrémité libre est connectée, ou glissée à travers ou aplatie à l'intérieur, et est bloquée par l'organe de blocage 70. Ledit organe de blocage 70 est fixé au cathéter 11 au niveau de l'extrémité proximale du cathéter par exemple de la valve d'instillation 26c de l'agent thérapeutique. Dans cette variante de réalisation, l'organe de blocage 70 est un boulon 71 ou une roue captive dans une cage 72 ou un winch 73 ou une épingle 74 ou une plaque circulaire perforée 75 ou un crochet 77 ou composé de bandes auto-agrippantes 76.

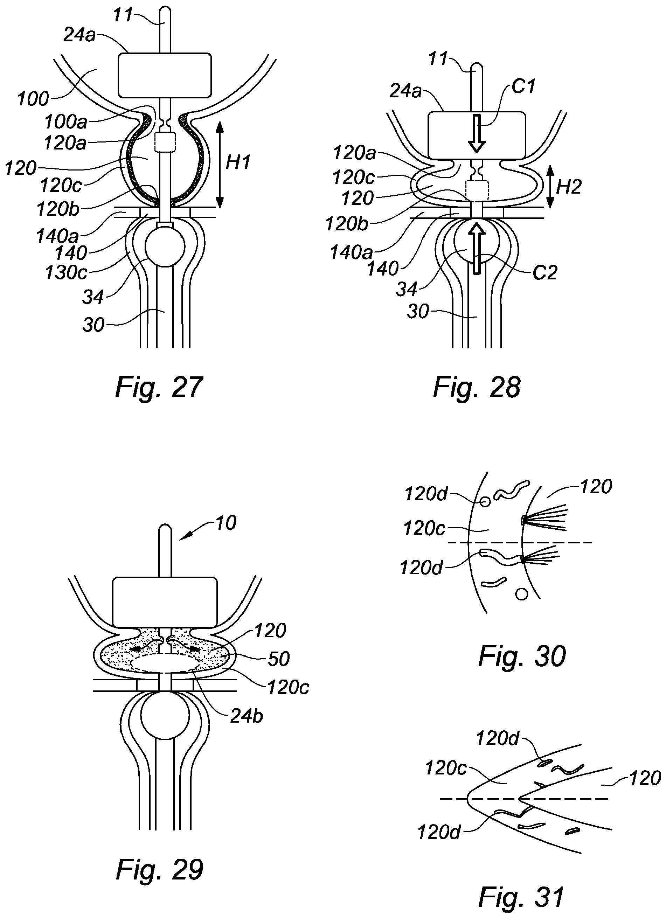

Les figures 27 à 31 illustrent la mise en place et le fonctionnement du dispositif hémostatique 10 selon la première variante du deuxième mode de réalisation comme illustré figure 4. Les structures anatomiques présentées dans ces figures sont les suivantes: la vessie 100, le col de la vessie 100a, la cavité prostatique 120, la paroi de la cavité prostatique 120c, le sphincter urinaire 140, le diaphragme périnéal 140a et l'urètre bulbaire 130c.

En figure 27, le dispositif hémostatique 10 se trouve dans un état non activé. Après insertion du dispositif hémostatique 10, le premier ballonnet 24a du cathéter 11 a été gonflé à l'intérieur de la vessie 100 et le troisième ballonnet 34 du dispositif de blocage a été gonflé à l'intérieur de l'urètre bulbaire 130c. H1 est la hauteur de la cavité prostatique 120, c'est-à-dire la distance entre l'orifice supérieur 120a de la cavité prostatique 120 et l'orifice inférieur 120b, situé juste au-dessus du sphincter strié 140 incorporé au plan du diaphragme périnéal 140a. La zone de saignement est représentée par un trait épais continu couvrant l'intérieur de la paroi 120c de la cavité prostatique 120. Le suintement hémorragique des petits vaisseaux et des capillaires de ces deux orifices est sous-estimé pendant et après la chirurgie. Ce suintement hémorragique est difficile à voir et à contrôler pendant l'opération. En effet, ce saignement peu abondant mais permanent est lavé continuellement par le liquide d'irrigation perfusé sous pression pour éclaircir la vision du champ opératoire ; ceci explique qu'il n'est pas ou mal contrôlé pendant l'opération, alors qu'à l'opposé un saignement en jet provenant de la section de plus gros vaisseaux de la paroi prostatique 120c est clairement visible et est plus facilement contrôlé pendant l'opération. De plus, la coagulation du suintement hémorragique provenant de l'orifice inférieur de la cavité 120b expose au risque de lésion du sphincter strié 140 situé juste en dessous par diffusion de l'énergie transmise par l'instrument pour coaguler la circonférence de l'orifice inférieur de la cavité 120b.

La figure 28 montre l'effet de la force de compression C1 dirigée vers le bas et exercée par le premier ballonnet 24a du cathéter 11 contre l'orifice supérieur 120a de la cavité prostatique 120 et l'effet de la force de compression C2 opposée dirigée vers le haut du troisième ballonnet 34 du tube de blocage 30 contre le sphincter strié 140 et à travers celui-ci contre l'orifice inférieur 120b de la cavité 120. Cette manœuvre entraîne une compression longitudinale de la cavité prostatique 120 située entre le premier ballonnet 24a et le troisième ballonnet 34 du dispositif de blocage 12 ainsi que la réduction significative de la hauteur de la cavité prostatique 120 de H1 à H2. Les flèches représentent respectivement les forces longitudinales antagonistes de compression C1 et C2. Afin de maintenir la compression de la cavité prostatique 120 par le premier ballonnet 24a et le troisième ballonnet 34 du dispositif de blocage 12, l'organe de blocage 40 (non représenté sur la figure 28) est activé. Cette manœuvre bloque la position relative du premier ballonnet 24a du cathéter 11 et du troisième ballonnet 34 du dispositif de blocage 12 dans un état activé. La cavité prostatique 120 est compressée, sa paroi 120c est pliée et ses deux orifices supérieur 120a et inférieur 120b sont fermés de façon étanche.

La compression et la fermeture de la cavité sont standardisées et reproductibles du fait des caractéristiques de l'organe de blocage 40 comme décrit ci-dessus. La reproductibilité est assurée, quelle que soit la taille de la prostate avant chirurgie et la taille de la cavité après chirurgie. La compression et la plicature de la cavité prostatique diminuent le volume de la cavité et de ce fait le volume du caillot qui peut se former à l'intérieur avant instillation de l'agent thérapeutique dans la cavité. Comme déjà précisé plus haut, plus le caillot est petit, plus les risques liés à la fibrinolyse seront diminués et meilleure sera l'hémostase.

Comme illustré en figure 30, avant compression, la paroi de la cavité a une épaisseur normale ; la lumière des vaisseaux 120d est normalement ouverte. En cas de section de vaisseaux 120d sur le versant interne de la paroi de la cavité prostatique 120c, les vaisseaux 120d peuvent saigner dans la cavité 120, lorsque leur contrôle a été insuffisant pendant l'opération.

Comme illustré en figure 31, après compression, la paroi de la cavité 120c est pliée et aplatie; la lumière des vaisseaux 120d est également pliée, aplatie et obturée. Cet effet mécanique facilite l'hémostase des vaisseaux 120d coupés sur le versant interne de la paroi da la cavité 120c.

Comme illustré en figure 29, l'agent thérapeutique est instillé à l'intérieur de la cavité prostatique 120 compressée et hermétiquement fermée. Selon la configuration du cathéter, le gonflage du deuxième ballonnet 24b représenté par une ligne pointillée à l'intérieur de la cavité prostatique 120 est réalisé. Le gonflage du deuxième ballonnet 24b facilite la diffusion de l'agent thérapeutique 50 contre la face interne de la paroi 120c de la cavité prostatique 120. L'effet biochimique de l'agent thérapeutique complète l'hémostase.

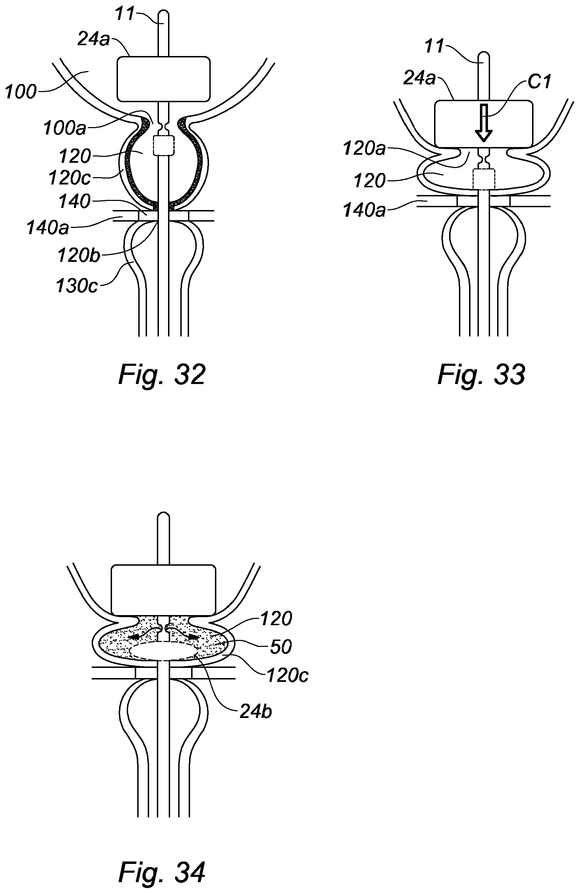

Les figures 32 à 34 illustrent la mise en place et le fonctionnement du dispositif hémostatique 10 selon la deuxième variante du deuxième mode de réalisation. La deuxième variante est caractérisée par le deuxième exemple du dispositif de blocage visible en figure 11 ou sa variante visible figure 12. Les structures anatomiques présentées dans ces figures 32 à 34 sont les suivantes : la vessie 100, le col de la vessie 100a, la cavité prostatique 120, la paroi de la cavité prostatique 120c, le sphincter urinaire 140, le diaphragme périnéal 140a et l'urètre bulbaire 130c.

Comme illustré en figure 32, les muscles et fascia du diaphragme périnéal 140a du plancher périnéal, structure anatomique fixe et résistante, jouent le rôle du troisième ballonnet 34 du tube de blocage 30 tel que préalablement décrit dans le premier exemple de réalisation du dispositif de blocage dans les figures 27 à 29.. Comme illustré dans les figures 32 à 34, les étapes de la technique de compression et d'occlusion de la cavité 120 avec ce deuxième exemple de réalisation du dispositif de blocage sont légèrement différentes de celles décrites pour le premier exemple de réalisation du dispositif de blocage dans les figures 27 à 29. Comme illustré en figure 32, après insertion du dispositif, le premier ballonnet 24a du cathéter 11 est gonflé à l'intérieur de la vessie 100. Le dispositif se trouve dans un état non activé.

Comme représenté en figure 33, le premier ballonnet 24a du cathéter 11 est tiré contre l'orifice supérieur 120a de la cavité prostatique 120. La cavité prostatique 120 est comprimée contre le diaphragme périnéal 140a. La flèche C1 représente la force de compression exercée contre la force de résistance passive antagoniste du diaphragme périnéal 140a. Pour maintenir la compression de la cavité prostatique 120, l'organe de blocage 70 est activé (non représenté sur la figure). Cette manœuvre fixe la position relative du premier ballonnet 24a du cathéter 11 contre le diaphragme périnéal 140a. Le dispositif hémostatique est alors dans un état activé. La cavité prostatique 120 compressée et pliée est fermée de façon étanche.

Comme illustré en figure 34, l'agent thérapeutique est instillé à l'intérieur de la cavité prostatique 120 compressée et fermée de façon étanche. En fonction de la configuration du cathéter, le deuxième ballonnet 24b, représenté par une ligne pointillée sur la figure, est gonflé à l'intérieur de la cavité prostatique 120. Le gonflage du deuxième ballonnet 24b facilite la propagation de l'agent thérapeutique 50 et son contact avec la face interne de la paroi 120c de la cavité prostatique 120.

Le dispositif hémostatique selon un troisième mode de réalisation va maintenant être décrit notamment en référence aux figures 13 à 17.

Le troisième mode de réalisation du dispositif hémostatique 10 diffère de la première variante et de la deuxième variante du deuxième mode de réalisation, en ce qu'il comprend deux canaux supplémentaires : un canal de drainage de l'urine 25a et un canal d'irrigation 25d.

Le cathéter 11 du dispositif hémostatique 10 selon le troisième mode comprend un canal de drainage d'urine 25a. Le cathéter 11 comprend en outre, un orifice de drainage 23 destiné à être positionné dans la vessie. L'orifice de drainage 23 est ménagé sur le corps du cathéter 11 entre l'extrémité distale 22c et le premier ballonnet 24a du cathéter, au niveau d'une portion 20b de l'extrémité distale 22c. L'orifice 23 draine l'urine par le canal central 25a.

Par ailleurs, le cathéter 11 comprend un premier cône 28a, configuré pour permettre l'évacuation de l'urine provenant du canal de drainage d'urine 25a, à partir de l'orifice de drainage 23. Le premier cône 28a est destiné à s'adapter à son homologue mâle d'une tubulure d'un sac collecteur de l'urine (non représenté).

Le cathéter 11 comprend un canal d'irrigation 25d configuré pour permettre la perfusion d'un liquide d'irrigation, si nécessaire, pour permettre le lavage de la vessie et la dilution du sang. Le canal d'irrigation 25d chemine dans la paroi 21 du cathéter 11 et se termine par un orifice d'irrigation 27c. L'orifice d'irrigation 27c est situé à la partie distale 20b du cathéter, entre l'orifice de drainage 23 mentionné ci-dessus et le premier ballonnet 24a situé à la partie distale 20b dudit cathéter.

Le cathéter 11 comprend en outre un deuxième cône 28b configuré pour permettre la perfusion d'un liquide d'irrigation. Le deuxième cône 28b est situé à l'extrémité proximale 20a du cathéter 11 et permet la connexion avec le cône homologue mâle d'une tubulure d'un sac contenant la solution d'irrigation (non représenté).

Dans une variante non représentée, le cathéter 11 comprend une spirale (non représentée sur la figure) métallique ou en nylon configurée pour renforcer la paroi 21 du cathéter 11 et empêcher l'occlusion de sa lumière, à l'endroit où le cathéter 11 est soumis à une forte angulation voisine de 90 degrés, comme à la jonction entre le segment bulbaire 130c de l'urètre et les segments membraneux et prostatique 130b-130a, comme visible en figure 21.

Le dispositif hémostatique 10 selon le troisième mode de réalisation illustré figures 13 à 17 comprend un dispositif de blocage 12 selon le premier exemple de réalisation comme illustré et décrit en référence à la figure 6.

Le cathéter 11 du dispositif hémostatique 10 selon le troisième mode de réalisation va maintenant être décrit. Le cathéter 11 présente des fonctionnalités de drainage de l'urine et d'irrigation de la vessie. Ainsi, les caractéristiques dépendantes de ces fonctionnalités sont ajoutées : le canal de drainage 25a, le canal d'irrigation 25d, l'orifice de drainage 23, l'orifice d'irrigation 27c, le premier cône 28a, et le deuxième cône 28b. La partie du cathéter 11 située entre le premier ballonnet 24a et l'extrémité distale 22c n'est pas borgne mais perméable et en communication fluidique avec l'orifice de drainage 23 et le canal de drainage 25a.

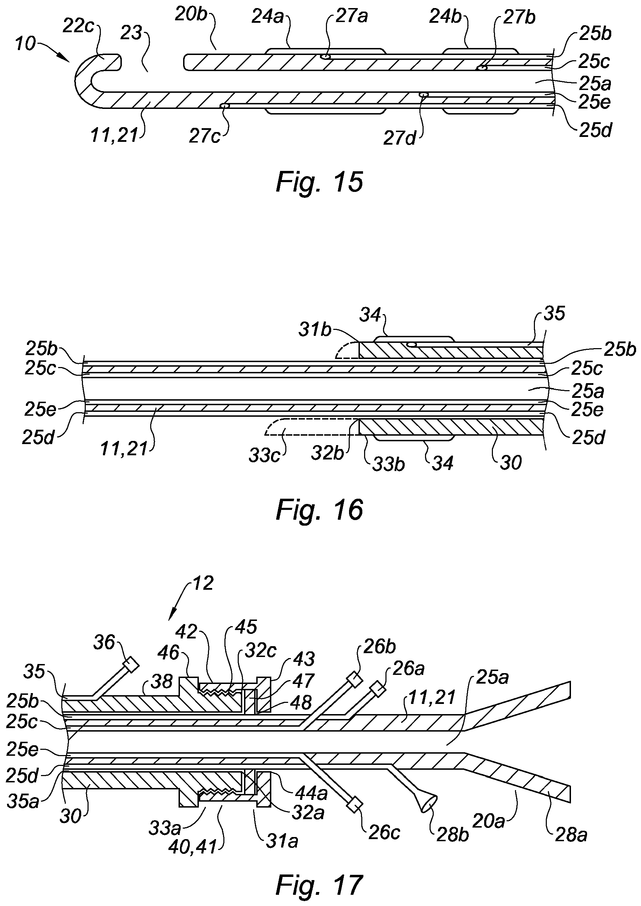

A l'intérieur du corps 20 du cathéter 11 il y a donc deux canaux en plus du canal unique 25b pour le gonflage par la valve 26a du premier ballonnet 24a selon le premier mode de réalisation du dispositif hémostatique (figure 1), ou en plus des deux canaux, ledit canal 25b et le deuxième canal 25c pour le gonflage par la valve 26b du deuxième ballonnet 24b selon la variante du premier mode (figure 2) ou encore en plus des trois canaux, lesdits canaux 25b, 25c et le canal 25e pour l'instillation par la valve 26c de l'agent thérapeutique 50 selon la première variante du deuxième mode (figures 4 à 5 et 7A à 12). Le canal 25e du cathéter selon le troisième mode de réalisation du dispositif hémostatique a un diamètre nettement inférieur à celui du canal 25e du cathéter selon le deuxième mode de réalisation du dispositif hémostatique 10. L'agent thérapeutique 50 sort par un seul orifice 27d situé en dessous du premier ballonnet 24a comme illustré figure 15. La taille de l'orifice unique 27d est inférieure à celle de chacun des deux orifices 27d du cathéter selon le deuxième mode de réalisation du cathéter préalablement illustré figure 8.

Le dispositif hémostatique 10 selon le troisième mode de réalisation comprend un dispositif de blocage 12 soit selon le premier exemple de réalisation (figure 13), soit selon le deuxième exemple de réalisation (figure 18) ou de sa variante (figure 20A). Le deuxième exemple de réalisation du dispositif de blocage et sa variante ont déjà été illustrés dans les figures 11 et 12 et ont déjà été décrits dans les paragraphes traitant du deuxième mode de réalisation du dispositif hémostatique 10 selon l'invention.

Comme expliqué plus haut, l'organe de blocage 70, le moyen d'arrimage 80 et leurs variantes respectives telles que décrites en figures 11 et 12 pour l'utilisation du dispositif hémostatique 10 selon le deuxième mode de réalisation, peuvent être adaptés au dispositif hémostatique 10 selon le troisième mode de réalisation. Comme illustré aux figures 18 à 20B, l'organe de blocage 70 peut être un boulon 71 ou une roue captive dans une cage 72 ou un winch 73 ou une épingle 74 ou une plaque circulaire perforée 75 ou une ou plusieurs bandes Velcro 76 ou un crochet 77.

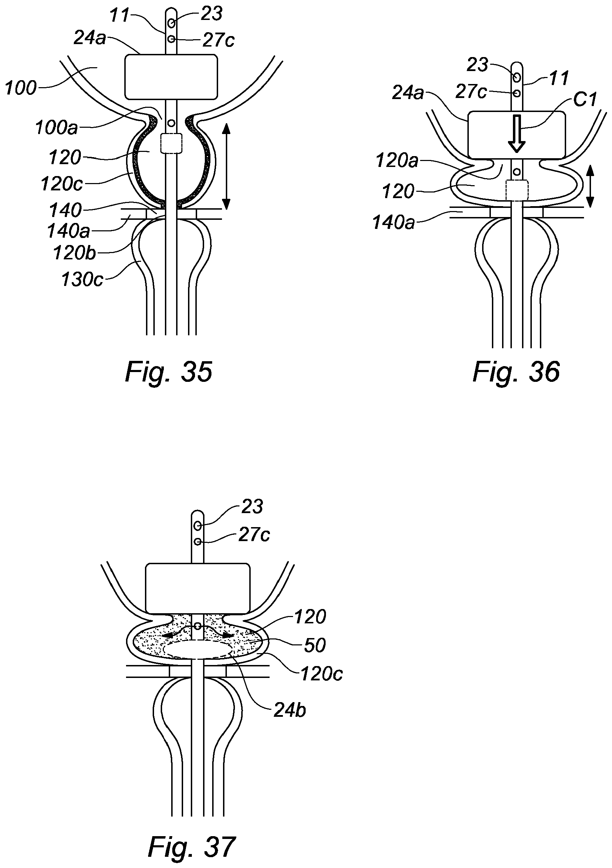

Les figures 35 à 37 illustrent la mise en place et le fonctionnement du dispositif hémostatique 10 selon une des deux variantes (figures 18 et 19) du troisième mode de réalisation préalablement illustré aux figures 13 à 17. Les structures anatomiques présentées dans ces figures sont les suivantes : la vessie 100, le col de la vessie 100a, la cavité prostatique 120, la paroi de la cavité prostatique 120c, le sphincter urinaire 140, le diaphragme périnéal 140a et l'urètre bulbaire 130c.

Comme représenté en figure 35, après insertion du dispositif hémostatique 10, le premier ballonnet 24a est gonflé à l'intérieur de la vessie 100. Le dispositif hémostatique 10 se trouve dans un état non activé.

Comme illustré en figure 36, le premier ballonnet 24a est tiré contre l'orifice supérieur 120a de la cavité prostatique 120. La cavité prostatique 120 est comprimée contre le diaphragme périnéal 140a. La flèche C1 représente la force de compression exercée contre la force de résistance passive antagoniste du diaphragme périnéal 140a. Pour maintenir la compression de la cavité prostatique 120, l'organe de blocage 70 est activé (non représenté). Cette manœuvre fixe la position relative du premier ballonnet 24a contre le diaphragme périnéal 140a dans un état de tension. Le dispositif hémostatique 10 est activé : la cavité prostatique 120 est compressée, pliée et fermée de façon étanche, standardisée et reproductible.

Comme illustré en figure 37, l'agent thérapeutique 50 est instillé à l'intérieur de la cavité prostatique 120 compressée et fermée de façon étanche.

En fonction de la configuration du cathéter, le deuxième ballonnet 24b, représenté par une ligne pointillée sur la figure, est gonflé à l'intérieur de la cavité prostatique 120. Le gonflage du deuxième ballonnet 24b facilite la propagation de l'agent thérapeutique 50 et son contact avec la face interne de la paroi 120c de la cavité prostatique 120.

Bien entendu, l'invention n'est pas limitée aux modes de réalisation décrits et représentés aux figures annexées. Des modifications restent possibles, notamment du point de vue de la constitution des divers éléments ou par substitution d'équivalents techniques, sans sortir pour autant du domaine de protection de l'invention.

## Revendications

1. Dispositif hémostatique (10) pour le traitement du saignement postopératoire de la cavité prostatique (120) comportant au moins un cathéter (11) urétral comprenant :
• un corps (20) comportant un premier canal (25b),
• un premier ballonnet (24a) gonflable ménagé sur ledit corps (20), le premier ballonnet (24a) étant en connexion fluidique avec le premier canal (25b), le premier ballonnet (24a) étant destiné à être placé dans la vessie (100) contre le col de la vessie (100a) et étant configuré pour exercer une pression déterminée à l'état gonflé sur la cavité prostatique (120) de manière à obturer et isoler la cavité prostatique (120), à réduire le volume de la cavité prostatique (120) et à occlure les vaisseaux de la paroi de la cavité prostatique (120),
**caractérisé en ce que** le premier ballonnet (24a) présente une forme globalement cylindrique invariable, un volume à gonfler invariable, et est rigide lorsqu'il est gonflé à une pression de l'ordre de 2 bars de telle sorte que ledit premier ballonnet (24a) à l'état gonflé ne se déforme pas ni se conforme à la forme des tissus et **en ce que** le dispositif hémostatique (10) comprend en outre au moins un dispositif de blocage (12) de position configuré pour figer la position et la tension du cathéter(1) dans l'urètre (130), le dispositif de blocage (12) étant relié directement ou indirectement au cathéter(11) .

2. Dispositif hémostatique selon la revendication 1, dans lequel le premier ballonnet (24a) est obtenu dans du nylon.

3. Dispositif hémostatique selon l'une quelconque des revendications 1 ou 2, dans lequel le corps (20) comprend au moins un deuxième canal (25c), un deuxième ballonnet (24b), ledit deuxième canal (25c) étant relié fluidiquement au deuxième ballonnet (24b), le deuxième ballonnet (24b) étant conformé pour, à l'état gonflé, s'étendre dans la cavité prostatique (120) et dans lequel le deuxième ballonnet (24b) est configuré pour se conformer à la forme des tissus et particulièrement à la forme irrégulière de la cavité prostatique dans laquelle il est gonflé.

4. Dispositif hémostatique selon l'une quelconque des revendications 1 à 3, dans lequel le cathéter (11) comporte un troisième canal (25e) comprenant un orifice de sortie (27d), et un orifice d'entrée, le troisième canal (25e) étant configuré pour acheminer depuis l'orifice d'entrée un agent thérapeutique (50), préférentiellement un ensemble de peptides de synthèse auto-agrégeant ou un agent coagulant ou un agent de comblement, vers l'orifice de sortie (27d), l'orifice de sortie (27d) étant destiné à déboucher dans la cavité prostatique (120) en état d'utilisation.

5. Dispositif hémostatique selon l'une quelconque des revendications 1 à 4, comprenant au moins un quatrième canal (35), un troisième ballonnet (34), ledit quatrième canal (35) étant relié fluidiquement au troisième ballonnet (34), le troisième ballonnet (34) étant conformé pour, à l'état gonflé, s'étendre dans l'urètre bulbaire (130c).

6. Dispositif hémostatique selon la revendication 5, dans lequel le troisième ballonnet (34) est ménagé sur le dispositif de blocage (12) du dispositif hémostatique (10).

7. Dispositif hémostatique selon l'une quelconque des revendications 1 à 6, dans lequel le corps du cathéter comprend un canal de drainage de l'urine (25a).

8. Dispositif hémostatique selon l'une quelconque des revendications 1 à 7, dans lequel le corps du cathéter comprend un canal d'irrigation (25d) d'un liquide d'irrigation.

9. Dispositif hémostatique selon l'une quelconque des revendications 1 à 8, dans lequel le dispositif de blocage (12) est relié indirectement au cathéter (11) par un moyen de raccordement (60).

10. Dispositif hémostatique selon l'une quelconque des revendications 1 à 9, dans lequel le premier ballonnet (24a) présente un revêtement hydrophile, au moins sur la moitié inférieure proximale dudit premier ballonnet (24a), afin de permettre une fermeture étanche de l'orifice supérieur de la cavité prostatique (120).

## Patentansprüche

1. Hämostatische Vorrichtung (10) zur Behandlung der postoperativen Blutung der Prostatahöhle (120), aufweisend mindestens einen Harnleiterkatheter (11), umfassend:
• einen Körper (20), der einen ersten Kanal (25b) aufweist,
• einen ersten aufblasbaren Ballon (24a), der auf dem Körper (20) eingerichtet ist, wobei der erste Ballon (24a) in Fluidverbindung mit dem ersten Kanal (25b) ist, wobei der erste Ballon (24a) zum Platzieren in der Harnblase (100) gegen den Hals der Harnblase (100a) bestimmt ist und ausgelegt ist, um im aufgeblasenen Zustand einen bestimmten Druck auf die Prostatahöhle (120) derart auszuüben, dass die Prostatahöhle (120) verschlossen und isoliert wird, das Volumen der Prostatahöhle (120) reduziert und die Gefäße der Wand der Prostatahöhle (120) verschlossen werden,
**dadurch gekennzeichnet, dass** der erste Ballon (24a) eine global zylindrische unveränderliche Form, ein unveränderliches aufzublasendes Volumen aufweist und starr ist, wenn er mit einem Druck von zirka 2 bar aufgeblasen ist, dass sich der erste Ballon (24a) in aufgeblasenem Zustand nicht verformt noch sich an die Form der Gewebe anpasst und dass die hämostatische Vorrichtung (10) ferner mindestens eine Positions-Arretiervorrichtung (12) umfasst, die ausgelegt ist, um die Position und die Spannung des Katheters (1) im Harnleiter (130) festzustellen, wobei die Arretiervorrichtung (12) direkt oder indirekt mit dem Katheter (11) verbunden ist.

2. Hämostatische Vorrichtung nach Anspruch 1, wobei der erste Ballon (24a) aus Nylon hergestellt ist.

3. Hämostatische Vorrichtung nach einem der Ansprüche 1 oder 2, wobei der Körper (20) mindestens einen zweiten Kanal (25c), einen zweiten Ballon (24b) umfasst, wobei der zweite Kanal (25c) mit dem zweiten Ballon (24b) fluidisch verbunden ist, wobei der zweite Ballon (24b) ausgebildet ist, um sich im aufgeblasenen Zustand in der Prostatahöhle (120) zu erstrecken und wobei der zweite Ballon (24b) ausgelegt ist, um sich an die Form der Gewebe und insbesondere an die unregelmäßige Form der Prostatahöhle anzupassen, in der er aufgeblasen ist.

4. Hämostatische Vorrichtung nach einem der Ansprüche 1 bis 3, wobei der Katheter (11) einen dritten Kanal (25e) aufweist, der eine Auslassöffnung (27d) und eine Einlassöffnung umfasst, wobei der dritte Kanal (25e) ausgelegt ist, um ab der Einlassöffnung ein therapeutisches Mittel (50), vorzugsweise eine Gruppe selbstaggregierender Synthesepeptide oder ein koagulierendes Mittel oder ein Verschlussmittel, zu der Auslassöffnung (27d) zu befördern, wobei die Auslassöffnung (27d) bestimmt ist, im Benutzungszustand in der Prostatahöhle (120) auszumünden.

5. Hämostatische Vorrichtung nach einem der Ansprüche 1 bis 4, umfassend mindestens einen vierten Kanal (35), einen dritten Ballon (34), wobei der vierte Kanal (35) mit dem dritten Ballon (34) fluidisch verbunden ist, wobei der dritte Ballon (34) ausgebildet ist, um sich im aufgeblasenen Zustand im bulbären Harnleiter (130c) zu strecken.

6. Hämostatische Vorrichtung nach Anspruch 5, wobei der dritte Ballon (34) auf der Arretiervorrichtung (12) der hämostatischen Vorrichtung (10) eingerichtet ist.

7. Hämostatische Vorrichtung nach einem der Ansprüche 1 bis 6, wobei der Körper des Katheters einen Urin-Drainagekanal (25a) umfasst.

8. Hämostatische Vorrichtung nach einem der Ansprüche 1 bis 7, wobei der Körper des Katheters einen Spülkanal (25d) einer Spülflüssigkeit umfasst.

9. Hämostatische Vorrichtung nach einem der Ansprüche 1 bis 8, wobei die Arretiervorrichtung (12) mit dem Katheter (11) indirekt über ein Anschlussmittel (60) verbunden ist.

10. Hämostatische Vorrichtung nach einem der Ansprüche 1 bis 9, wobei der erste Ballon (24a) eine hydrophile Beschichtung mindestens auf der unteren proximalen Hälfte des ersten Ballons (24a) aufweist, um einen dichten Verschluss der oberen Öffnung der Prostatahöhle (120) zu gestatten.

## Claims

1. An hemostatic device (10) for the treatment of postoperative bleeding from the prostatic cavity (120) including at least one urethral catheter (11) comprising:
• a body (20) including a first channel (25b),
• a first inflatable balloon (24a) formed on said body (20), the first balloon (24a) being in fluid connection with the first channel (25b), the first balloon (24a) being intended to be placed in the bladder (100) against the bladder neck (100a) and being configured to exert a determined pressure in the inflated state on the prostatic cavity (120) so as to close and isolate the prostatic cavity (120), reduce the volume of the prostatic cavity (120) and to occlude the vessels of the wall of the prostatic cavity (120),
**characterized in that** the first balloon (24a) has an invariable generally cylindrical shape, an invariable volume to be inflated, and is rigid when inflated to a pressure of about 2 bars such that said first balloon (24a) in the inflated state is not deformed nor compliant with the shape of the tissues and **in that** the hemostatic device (10) further comprises at least one position blocking device (12) configured to freeze the position and the tension of the catheter (1) in the urethra (130), the blocking device (12) being directly or indirectly connected to the catheter (11).

2. The hemostatic device according to claim 1, wherein the first balloon (24a) is made of nylon.

3. The hemostatic device according to any one of claims 1 or 2, wherein the body (20) comprises at least a second channel (25c), a second balloon (24b), said second channel (25c) being fluidly connected to the second balloon (24b), the second balloon (24b) being shaped to, in the inflated state, extend into the prostatic cavity (120) and wherein the second balloon (24b) is configured to be compliant with the shape of the tissues and in particular with the irregular shape of the prostatic cavity in which it is inflated.

4. The hemostatic device according to any one of claims 1 to 3, wherein the catheter (11) includes a third channel (25e) comprising an outlet orifice (27d), and an inlet orifice, the third channel (25e) being configured to convey, from the inlet orifice, a therapeutic agent (50), preferably a set of self-aggregating synthetic peptides or a coagulating agent or a filling agent, towards the outlet orifice (27d), the outlet orifice (27d) being intended to open into the prostatic cavity (120) in use.

5. The hemostatic device according to any one of claims 1 to 4, comprising at least a fourth channel (35), a third balloon (34), said fourth channel (35) being fluidly connected to the third balloon (34), the third balloon (34) being shaped to, in the inflated state, extend into the bulbar urethra (130c).

6. The hemostatic device according to claim 5, wherein the third balloon (34) is formed on the blocking device (12) of the hemostatic device (10).

7. The hemostatic device according to any one of claims 1 to 6, wherein the body of the catheter comprises a urine drainage channel (25a).

8. The hemostatic device according to any one of claims 1 to 7, wherein the body of the catheter comprises an irrigating channel (25d) for irrigating an irrigation liquid.

9. The hemostatic device according to any one of claims 1 to 8, wherein the blocking device (12) is indirectly connected to the catheter (11) by coupling means (60).

10. The hemostatic device according to any one of claims 1 to 9, wherein the first balloon (24a) has a hydrophilic coating, at least on the proximal lower half of said first balloon (24a), in order to allow a sealed closure of the upper orifice of the prostatic cavity (120).
